# EUROPEAN PATENT SPECIFICATION

(11) **EP 2 032 203 B1**
(45) Date of publication and mention of the grant of the patent: **23.10.2013**
(21) Application number: 07795734.8
(22) Date of filing: 05.06.2007
(51) Int. Cl.: A61N 1/36, A61F 2/00, A61N 1/05

(54) **ELECTRICAL MUSCLE STIMULATION TO TREAT FECAL INCONTINENCE AND/OR PELVIC PROLAPSE**
ELEKTRISCHE MUSKELSTIMULATION ZUR BEHANDLUNG VON FÄKALINKONTINENZ UND/ODER BECKENVORFALL
STIMULATION ELECTRIQUE DES MUSCLES POUR LE TRAITEMENT DE L'INCONTINENCE FECALE ET/OU DE L'APTOSE PELVIENNE

(30) Priority: 05.06.2006 US 803954 P; 16.06.2006 US 805036 P
(43) Date of publication of application: 11.03.2009
(73) Proprietor: AMS Research Corporation, Minnetonka, MN 55343 (US)
(72) Inventor: JIMENEZ, Jose, W., Minnetonka, MN 55343 (US); FERIANCEK, John, D., Minnetonka, MN 55343 (US)
(74) Representative: Vossius & Partner
(86) International application number: PCT/US2007/013190
(87) International publication number: WO 2007/145913

(56) References cited:
- WO-A-2005/122954
- US-A1- 2003 100 930
- US-A1- 2003 212 305
- US-A1- 2007 027 514

## Description

### TECHNICAL FIELD

The present invention relates generally to implantable medical devices, and specifically to implantable medical devices to relieve problems associated with fecal incontinence and related pelvic disorders.

### BACKGROUND

Fecal incontinence is a condition characterized by involuntary defecation or passage of feces through the anal canal due to injury to or weakness of one or more of the internal anal sphincter, the external anal sphincter, and the levator ani.

Urinary incontinence affects millions of people, causing discomfort and embarrassment, sometimes to the point of social isolation. In the United States, recent studies have shown that as many as 25 million persons, of whom approximately 85% are women, are affected by bladder control problems. Incontinence occurs in children and young adults, but the largest number affected are the elderly.

There are several major forms of urinary incontinence, including stress urinary incontinence, urge urinary incontinence, overflow urinary incontinence, and reflex urinary incontinence.

Stress incontinence is an involuntary loss of urine while doing physical activities, which put pressure on the abdomen. These activities include exercise, coughing, sneezing, laughing, lifting, or any body movement, which puts pressure on the bladder. Stress incontinence is typically associated with either or both of the following anatomical conditions:

Urethral hypermobility - Weakness of or injury to pelvic floor muscles causes the bladder to descend during abdominal straining or pressure, allowing urine to leak out of the bladder. This is the more common source of stress incontinence.

Intrinsic sphincter deficiency - In this condition, the urethral musculature is unable to completely close the urethra or keep it closed during stress.

Urge incontinence is the sudden urgent need to pass urine, and is caused by a sudden bladder contraction that cannot be consciously inhibited. This type of incontinence is not uncommon among healthy people, and may be linked to disorders such as infections that produce muscle spasms in the bladder or urethra. Urge incontinence may also result from illnesses that affect the central nervous system.

Overflow incontinence refers to leakage of urine that occurs when the quantity of urine exceeds the bladder's holding capacity, typically as a result of a blockage in the lower urinary tract.

Reflex incontinence is the loss of urine when the person is unaware of the need to urinate. This condition may result from nerve dysfunction, or from a leak in the bladder, urethra, or ureter.

Of the major forms of urinary incontinence listed above, the two most common are stress and urge. "Mixed incontinence" is a term used to describe the common phenomenon of the presence of stress and urge incontinence in the same patient.

A large variety of products and treatment methods are available for care of urinary or fecal incontinence. Most patients suffering from mild to moderate urinary or fecal incontinence use diapers or disposable absorbent pads. These products are not sufficiently absorbent to be effective in severe cases, are uncomfortable to wear, and can cause skin irritation as well as unpleasant odors. Other non-surgical products for controlling urinary incontinence include urethral inserts (or plugs), externally worn adhesive patches, and drugs.

Exercise and behavioral training are also effective in some cases in rehabilitating pelvic muscles and thus reducing or resolving urinary incontinence. Patients are taught to perform Kegel exercises to strengthen their pelvic muscles, which may be combined with electrical stimulation of the pelvic floor. Electromyographic biofeedback may also be provided to give the patients an indication as to the effectiveness of their muscular exertions. But retraining muscles is not possible or fully effective for most patients, particularly when there may be neurological damage or when other pathologies may be involved.

The InterStim^{®} System for Urinary Control sold by Medtronic, Inc., Fridley, Minnesota, comprises an implantable pulse generator (IPG), which is surgically implanted in the lower abdomen, and a medical electrical lead that extends from a connection with the IPG to exposed stimulation electrodes disposed adjacent the sacral nerve near the sacrum (the bone at the base of the spine) in a major surgical procedure - sometimes six hours under general anesthesia. The IPG continuously generates electrical stimulation pulses that are applied to the sacral nerve to control urinary voiding. The continuous electrical stimulation of the nerve has been found to control urge incontinence in some patients.

Various surgical procedures have been developed for bladder neck suspension, primarily to control urethral hypermobility by elevating the bladder neck and urethra. These procedures typically use bone anchors and sutures or slings to support the bladder neck. The success rates for bladder neck suspension surgery in controlling urinary leakage are typically approximately 60%-80%, depending on the patient's condition, the surgeon's skill, and the procedure that is used. The disadvantages of this surgical technique are its high cost, the need for hospitalization and long recovery period, and the frequency of complications.

For serious cases of intrinsic sphincter deficiency, artificial urinary sphincters have been developed. For example, the AMS 800 urinary sphincter, produced by America Medical Systems, Inc., of Minnetonka, Minnesota, includes a periurethral inflatable cuff, which is used to overcome urinary incontinence when the function of the natural sphincter is impaired. The cuff is coupled to a manually operated pump and a pressure regulator chamber, which are implanted in a patient's body together with the cuff. The cuff is maintained at a constant pressure of 60-80 cm of water, which is generally higher than the bladder pressure. To urinate, the patient releases the pressure in the cuff. Aspects of this system are described in U.S. Patent 4,222,377 to Burton.

The AMS Action® Neosphincter produced by American Medical Systems, Inc., of Minnetonka, Minnesota, is the only implantable sphincter available for the treatment of severe fecal incontinence. Using the AMS 800 technology, the Action® Neosphincter simulates normal anal sphincter function to give the patient control over defecation through a pressurized system. The Action® Neosphincter can be implanted in both men and women with a pressure regulating balloon placed in the prevesical space, the cuff implanted around a segment of the anal canal, and the pump positioned in either the scrotum or the labium.

These artificial urinary and fecal sphincters are of great benefit to certain patients but have some shortcomings. The constant concentric pressure that the periurethral cuff exerts on the urethra can result in impaired blood supply to tissue in the area, leading to tissue atrophy, urethral erosion and infection. Furthermore, the constant pressure in the cuff is not always sufficient to overcome transient increases in bladder pressure that may result from straining, coughing, laughing or contraction of the detrusor muscle. In such cases, urine leakage may result.

U.S. Patents 4,571,749 and 4,731,083 to Fischell describe an artificial sphincter device whose pressure can vary in response to changes in abdominal or intravesical (bladder) pressure. The device includes a periurethral cuff, subdermic pump, pressure regulator, and hydraulic pressure sensor.

U.S. Patent 3,628,538 to Vincent et al. describes external apparatus for stimulating a muscle based on an electromyogram (EMG) signal sensed in the muscle. If the signal is greater than a predetermined threshold value, a stimulator circuit applies a voltage to electrodes adjacent to the muscle. The apparatus is said to be particularly useful in overcoming incontinence, and the stimulation is preferably applied transcutaneously to the levator ani.

U.S. Patent 6,135,945 to Sultan describes apparatus for preventing uncontrolled discharge of urine from a patient's urethra. The apparatus includes an implantable pressure sensor for sensing intra-abdominal pressure, which generates a pressure signal in response to the sensed pressure. An actuating device is coupled to the pressure sensor, and generates an electrical signal in response to the pressure signal. A controller is coupled to the actuating device, and is configured to selectively compress the patient's urethra and thereby prevent incontinence.

Various types of electrodes have been proposed for applying electrical stimulation to pelvic muscles so as to prevent unwanted urine flow. For example, U.S. Patent 5,562,717 to Tippey et al. describes electrodes that are placed on the body surface, typically in the areas of the perineum and the sacrum, and are electrically actuated to control incontinence. U.S. Patent 4,785,828 to Maurer describes a vaginal plug having electrodes on an outer surface thereof. A pulse generator in the plug applies electrical pulses to the electrodes so as to constrict the patient's pelvic muscles and prevent urine flow. U.S. Patent 4,153,059 to Fravel et al. describes an intra-anal electrode, to which repetitive electrical pulses are applied in order to control urinary incontinence. U.S. Patent 4,106,511 to Erlandsson describes an electrical stimulator in the form of a plug for insertion into the vagina or the anus. U.S. Patent 3,866,613 to Kenny et al. describes a pessary ring having two electrodes thereon, which are energized to control incontinence. U.S. Patent 4,406,288 to Horwinski et al. describes apparatus for conditioning the pelvic floor musculature to reduce bladder contractility and relax the bladder, so as to prevent involuntary urinary loss.

U.S. Patent 4,580,578 to Barson describes a device for stimulating the sphincter muscles controlling the bladder. A supporting body is fitted into the patient's vulva between the labia, so that two electrodes attached to the supporting body contact the epidermal surface on either side of the external urethral orifice. Electrical pulses are applied to the electrodes to stimulate the region of the sphincter.

U.S. Patent 4,607,639 to Tanagho et al. describes a method for controlling bladder function by nerve stimulation, typically of a sacral nerve. The anatomical location of at least one nerve controlling the muscles for the bladder and/or its sphincter is identified, and an electrode is placed on the nerve to selectively stimulate the nerve for continence and evacuation purposes.

U.S. Patent 4,739,764 to Lue et al. describes a system for electrical stimulation of nerves in order to treat urinary incontinence, fecal incontinence, interstitial cystitis, and other pelvic pain syndromes.

U.S. Patent 6,240,315 to Mo et al. describes incontinence treatment apparatus, which includes a module for evaluating a recorded EMG signal.

U.S. Patent 5,484,445 to Knuth describes a system for anchoring a lead to the sacrum for purposes of long-term stimulation, typically for treatment of incontinence.

U.S. Patents 5,927,282 and 6,131,575 to Lenker et al. describe removable external closures for the urethra as means for relieving or mitigating incontinence problems.

U.S. Patent 6,002,964 to Feler et al. describes a method for managing chronic pelvic pain. The method includes techniques for positioning one or more stimulation leads within or about the sacrum to enable electrical energy to be applied to spinal nervous tissue, including nerve roots, in order to inhibit the transmission of pain signals.

An article by Fall et al., entitled, "Electrical stimulation in interstitial cystitis," Journal of Urology, 123(2), pp. 192-195, February, 1980, describes a study in which fourteen women with chronic interstitial cystitis were treated with long-term intravaginal or transcutaneous nerve stimulation. Clinical and urodynamic evaluations were performed after 6 months to 2 years. Improvement was not immediate, but required a considerable period of continuous, daily use of electrical stimulation.

An article by Zermann et al., entitled, "Sacral nerve stimulation for pain relief in interstitial cystitis," Urol. Int., 65(2), pp. 120-121, 2000, describes a case in which a 60-year-old woman was treated for severe interstitial cystitis pain using sacral nerve stimulation.

An article by Chai et al., entitled, "Percutaneous sacral third nerve root neurostimulation improves symptoms and normalizes urinary HB-EGF levels and antiproliferative activity in patients with interstitial cystitis," Urology, 55(5), pp. 643-646, May, 2000, notes: "A highly effective treatment for interstitial cystitis (IC) remains elusive.... Results suggest that permanent S3 PNS may be beneficial in treating IC."

An article by Caraballo et al., entitled, "Sacral nerve stimulation as a treatment for urge incontinence and associated pelvic floor disorders at a pelvic floor center: a follow-up study," Urology, 57(6 Suppl 1), p. 121, June, 2001, describes and presents the results of an additional study in which sacral nerve stimulation was applied in an effort to treat urinary incontinence.

PCT Patent Publication WO 00/19939, entitled, "Control of urge incontinence," which is assigned to the assignee of the present patent application, describes a device for treatment of urinary urge incontinence, in which imminent urge incontinence is sensed, and a pelvic nerve or muscle is stimulated to inhibit the flow.

PCT Patent Publication WO 00/19940, entitled, "Incontinence treatment device," which is assigned to the assignee of the present patent application, describes a device for treating urinary stress incontinence, in which imminent involuntary urine flow is sensed, and a pelvic nerve or muscle is stimulated to inhibit the flow.

A book entitled "Urinary Incontinence", edited by P. O'Donnell, Mosby Publishers, 1997, describes clinical aspects relating to the diagnosis and treatment of urinary incontinence.

As indicated in an article by Yamanishi et al., entitled "Electrical Stimulation for Stress Incontinence," Int Urogynecol J, (1998) 9:281-290 Springer-Verlag London, electrical stimulation of the levator ani was first tested by K.P.S. Caldwell in "The electrical control of sphincter incompetence." The Lancet, July 23, 1963, to treat fecal incontinence. Caldwell first employed an implantable stimulator coupled to electrodes sutured to the levator ani muscle at locations on either side of the pelvic floor using a retropubic approach or near the pudendal nerves via a perineal approach. The implantable stimulator was powered by radio frequency transmissions from an externally worn transmitter except during voiding or defecation. Yamanishi reports that the method is no longer used because of tissue reactions, surgical complications and technical problems.

An article by Yamamoto et al., entitled "Optimal parameters for effective electrical stimulation of the anal sphincters in a child with fecal incontinence: preliminary report," Pediatr Surg Int (1993) 8:132-137, describes determining optimal stimulation parameters for electrical stimulation of the anal sphincter of a child after abdominoperineal anorectoplasty for imperforate anus. In the testing procedure, intramuscular electrodes were implanted into the deep borders of the external anal sphincter to deliver stimulation to the striated external anal sphincter muscle fibers including the puborectalis. The pressure in the anal canal was measured as the amplitude (current intensity), width, and frequency of the alternating bi-directional biphasic pulses of regulated current generated buy an external stimulator were varied.

U.S. Patent No. 6,243,607 to Mintchev et al. describes arrays of stimulation electrodes supported on a mesh surrounding a portion of the GI tract and coupled to an IPG that synchronously applies pulses through the electrodes to the smooth muscle of the portion of the GI tract. Local contractions of the smooth muscle of the portion of the gastro-intestinal tract are artificially propagated distally through the electrode array in order to facilitate or aid at least a partial emptying of such portion. The local contractions are artificially propagated by phase locking or time shifting the electrical stimulus that is applied to the smooth muscle circumferentially about the portion at two or more locations. The portion of the gastro-intestinal tract may be comprised of the esophagus, the stomach, the small intestine, the large intestine, the anal sphincter and combinations thereof.

U.S. Patent Publication No. 2003/0028232 to Camps et al. discloses treating fecal incontinence by transplanting muscle from the patient's body around the anal sphincter and stimulating the muscle into contraction. A similar approach to treating fecal incontinence is suggested in the Yamamoto et al. article and to treating urinary incontinence is disclosed in U.S. Patent No. 6,659,936 to Furness et al., which is incorporated herein by reference.

It has also been suggested to implant fecal slings in the patient's body to support the anus and lower bowel or rectum in commonly assigned, copending U.S. Patent Application Publication No. 2007/0021650, entitled "Sling Assembly with Secure and Convenient Attachment". A fecal sling and an implantation method for implanting the fecal sling to extend around the anal sphincter to provide support and alleviate fecal incontinence are disclosed. In particular, a method is described for treating fecal incontinence in a patient comprising the steps of: providing a synthetic surgical mesh having first and second ends and a plurality of holes that are sized and shaped to afford tissue ingrowth, and a removable synthetic insertion sheath associated with the surgical mesh; providing a needle that is sized and shaped to be initially inserted through a suprapubic incision and to then emerge from at least one other incision, the needle having an insertion end and an end opposite the insertion end; providing a coupler having an axis, the coupler having a first end and a second end with surfaces for conveniently and securely connecting the coupler to the insertion end of the needle; creating at least one other incision; creating at least one suprapubic incision; passing the leading end of the needle initially through the suprapubic incision and then through the at least one other incision; then connecting the coupler to the needle by moving the coupler and insertion end of the needle together while the insertion end of the needle protrudes from the at least one other incision; implanting the sling by moving the leading end of the needle from the at least one other incision toward the suprapubic incision; and then removing the synthetic insertion sheath.

Methods and instruments for positioning a sling are also described in commonly assigned U.S. Patent Application Publication Nos. 2002/0161382 to Niesz et al., and 2004/0039453 to Anderson et al. and U.S. Patent Nos. 6,911,003 to Anderson et al. and 6,612,977 to Staskin et al. Transobturator or suprapubic methods, either up to or through the obturator foramen along with introducers/needles that can be used to place the sling in a desired location are described.

US-A-2003 100 930 discloses a device for treating fecal incontinence, having a device body supporting two stimulation electrodes, a lead and a tissue anchor.

WO-A-2005 122 954 and US-A-2003 212 305 disclose supportive slings for treating incontinence.

### SUMMARY

The invention is specified in the claims.

The preferred embodiments of the present invention provide apparatus for applying electrical stimulation to selected pelvic muscles to treat or alleviate or control fecal incontinence. Various aspects of the preferred embodiments of the invention may be used in combination or separately to apply electrical stimulation to one or more locations or positions in relation to an anal sphincter muscle comprising an internal anal sphincter surrounding the anus, an external anal sphincter surrounding the internal anal sphincter, a levator ani coupled to the external anal sphincter and perineal floor muscles around the anal orifice.

In certain embodiments, one or more stimulation electrode is supported on or part of or are associated with a mesh patch that is sized and shaped to fit in a particular location or position and adapted to assist in stabilizing or retaining the electrode(s) in the initial implantation position.

In other embodiments, one or more stimulation electrode is supported on or part of or are associated with a central support portion of an elongated fecal sling that is implanted in a tissue pathway disposing the central portion in a particular location or position, the fecal sling adapted to assist in stabilizing or retaining the electrode(s) in the initial implantation position and to apply supporting force to the anus.

In variations of these embodiments fixation mechanisms may be incorporated into or attached to the mesh patch ends or the sling ends to enable fixation in tissue or against membranes or fascia and to enable implantation employing single incision sling (SIS) implantation methods and instruments.

In further embodiments, one or more stimulation electrode is supported on or part of or are associated with a central aperture of a cuff of an artificial anal sphincter that is implanted about the anal cavity in a particular location or position, the cuff adapted to assist in stabilizing or retaining the electrode(s) in the initial implantation position and to be inflated to resist fecal incontinence and deflated to enable volitional voiding.

In certain embodiments, a sensor, e.g., a pressure sensor may be implanted in relation to the bowel or lower rectum or anus to detect filling and imminent bowel movement. The control unit logic and operating algorithm may employ the sensor signal to effect delivery of or modify delivery of electrical stimulation to the anus through the selected stimulation electrodes.

The expression "in relation to" or "in operative relation to" contemplates placing, positioning or locating stimulation electrodes adjacent to, within or upon one or more of the anal sphincter muscle comprising an internal anal sphincter surrounding the anus, an external anal sphincter surrounding the internal anal sphincter, a levator ani coupled to the external anal sphincter and perineal floor muscles around the anal orifice or placing, positioning or locating a sensor adjacent to, within or upon the wall of the rectum or bowel.

This summary has been presented here simply to point out some of the ways that the invention overcomes difficulties presented in the prior art and to distinguish the invention from the prior art and is not intended to operate in any manner as a limitation on the interpretation of claims that are presented initially in the patent application and that are ultimately granted.

The methods disclosed herein are provided to illustrate now the device according to the invention may be used. The methods do not form part of the invention.

### BRIEF DESCRIPTION OF THE DRAWINGS

The present invention will be more fully understood from the following detailed description of the preferred embodiments thereof, taken together with the drawings, in which:

Fig. 1A is a schematic, pictorial view of an implantable electronic stimulator device for prevention of mixed incontinence;

Fig. 1B is a schematic, pictorial view of an implantable electronic stimulator device for prevention of mixed incontinence;

Figs. 2A, 2B, 2C, 2D, 2E, 2F, and 2G show steps in an implantation procedure of a electronic stimulator device;

Fig. 2H is a schematic, partly sectional illustration showing implantation of the electronic stimulator device of Fig. 1A in the pelvis of a patient;

Fig. 2I is a schematic, partly sectional illustration showing implantation of the electronic stimulator device of Fig. 1A in the pelvis of a patient;

Fig. 3 is a schematic block diagram illustrating circuitry used in an implantable muscle stimulation device;

Fig. 4 is a schematic block diagram illustrating circuitry used in an implantable muscle stimulation device;

Fig. 5 is a schematic block diagram illustrating signal processing circuitry for analyzing EMG signals;

Figs. 6-9 are graphs showing simulated and measured signals, representative of different aspects of use of an implantable muscle stimulation device;

Fig. 10A is a schematic diagram of a pressure sensor;

Fig. 10B is a schematic, sectional illustration of the bladder of a patient, showing implantation therein of the pressure sensor of Fig. 10A;

Fig. 11 is a schematic, sectional illustration of the human pelvic region illustrating the anal sphincter and levator ani;

Fig. 12 is a schematic, sectional illustration of the human pelvic region illustrating locations or positions for installing stimulation electrodes for stimulating one or more of the internal and external anal sphincters, the levator ani and the perineal floor muscle;

Fig. 13 is a schematic, pictorial view of a bipolar implantable electronic stimulator device having stimulation electrodes associated with mesh patches for prevention of fecal incontinence;

Fig. 14 is a schematic, pictorial view of a unipolar implantable electronic stimulator device having stimulation electrodes associated with an elongated mesh patch for prevention of fecal incontinence;

Fig. 15 is a schematic, pictorial view of a unipolar implantable electronic stimulator device having stimulation electrodes associated with an elongated mesh sling for prevention of fecal incontinence;

Fig. 16 is a schematic, pictorial view of the unipolar implantable electronic stimulator device having stimulation electrodes associated with an elongated mesh sling implanted in a patient's body for prevention of fecal incontinence;

Fig. 17 is a schematic illustration of a fecal sling supporting stimulation electrodes with tissue anchors on the sling ends and an implantation tool enabling SIS implantation of the fecal sling; and

Fig. 18 is a schematic, pictorial view of a unipolar implantable electronic stimulator device having stimulation electrodes associated with the cuff of and artificial anal sphincter for prevention of fecal incontinence.

### DETAILED DESCRIPTION OF PREFERRED EMBODIMENTS

I. Overview of the prior art

A. General description of stimulator device

B. Sensing and control functions of the device

C. Signal processing

D. Power consumption control.

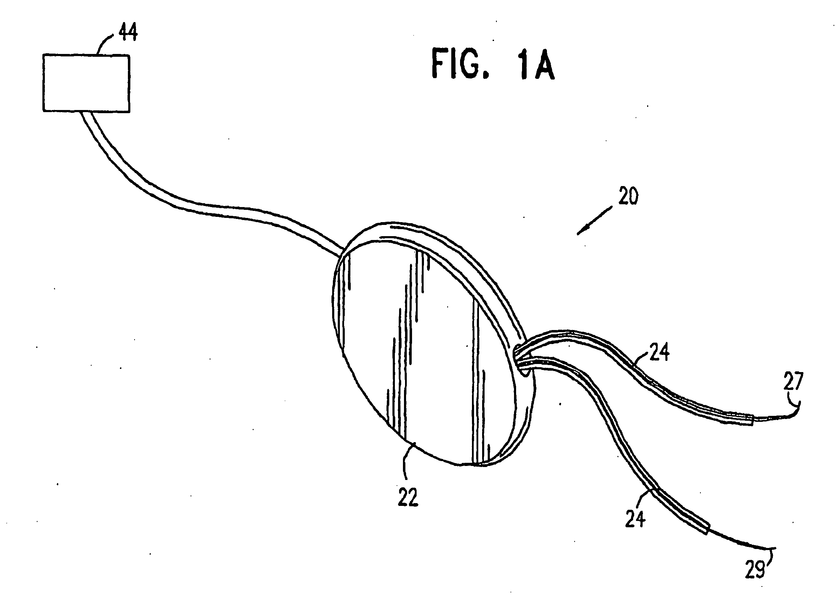

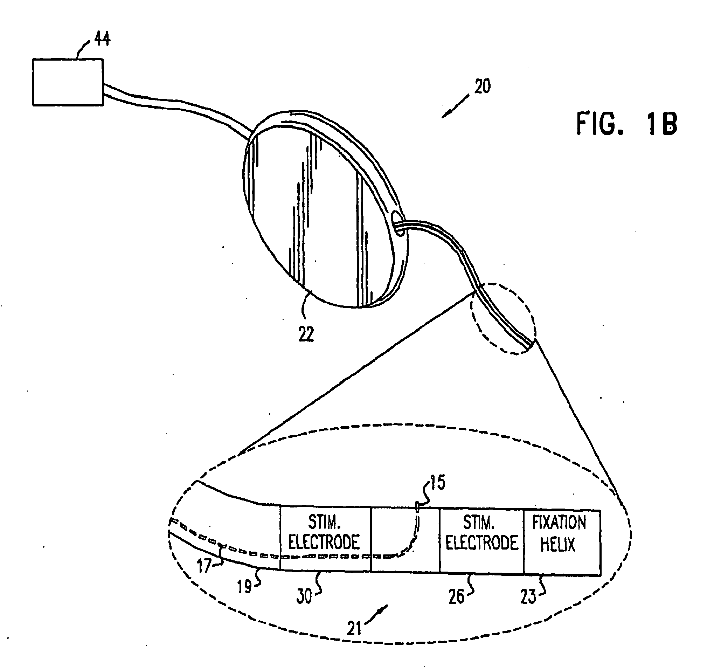

II. Detailed Description of Figures 1-10B

A. External elements of a stimulator device

B. Anatomical and surgical considerations

C. Signal processing

(i) hardware and algorithms

(ii) simulation of a typical EMG

(iii) experimentally measured EMG signals: Distinguishing incontinence from voluntary voiding

D. Muscle stimulation

E. Provision of power to the control unit

F. External communication with the control unit

G. Utilization of other sensors

H. Reduction of power consumption

III. Systems and methods for treating fecal incontinence and related pelvic floor disorders

I. Overview of the prior art

A. General description of stimulator device

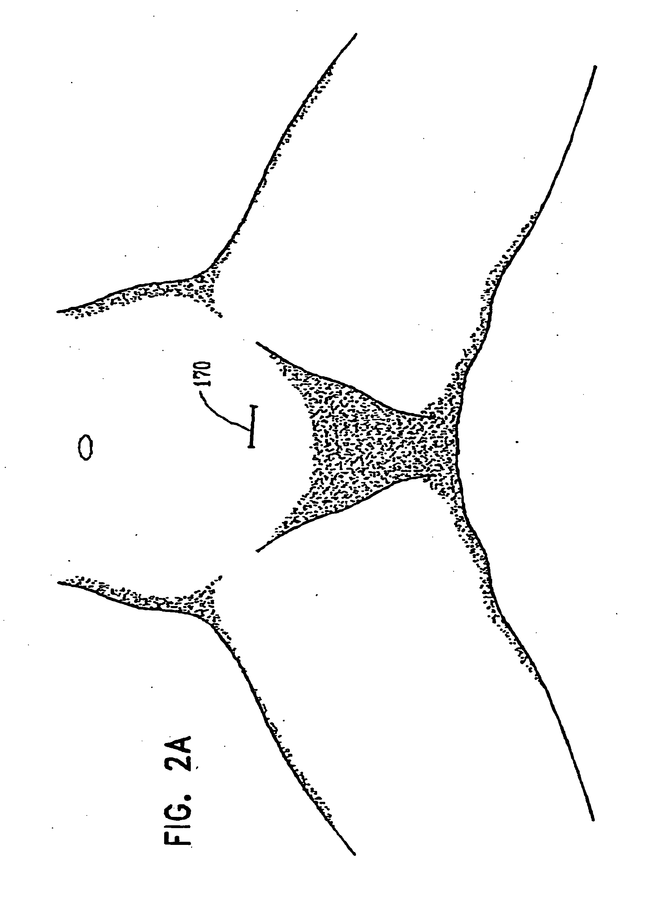

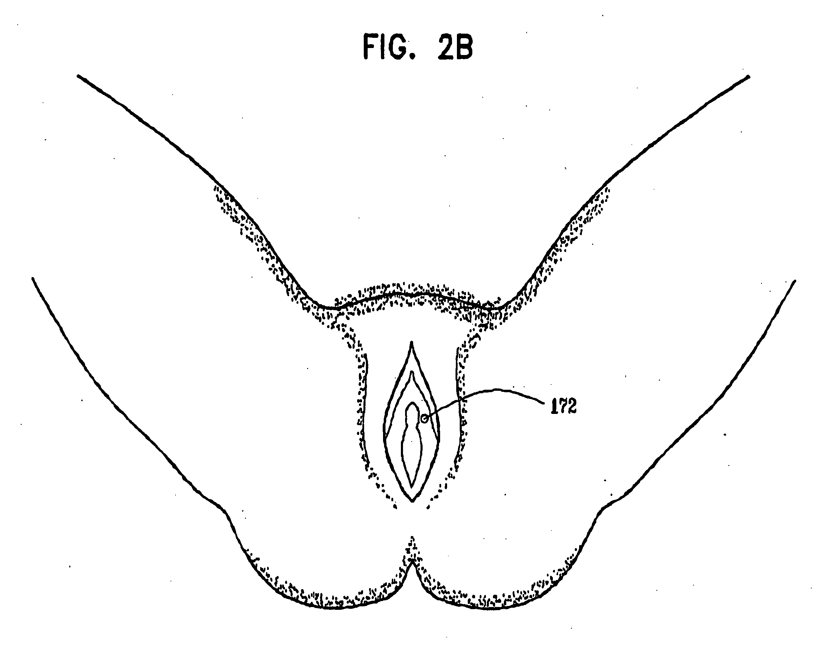

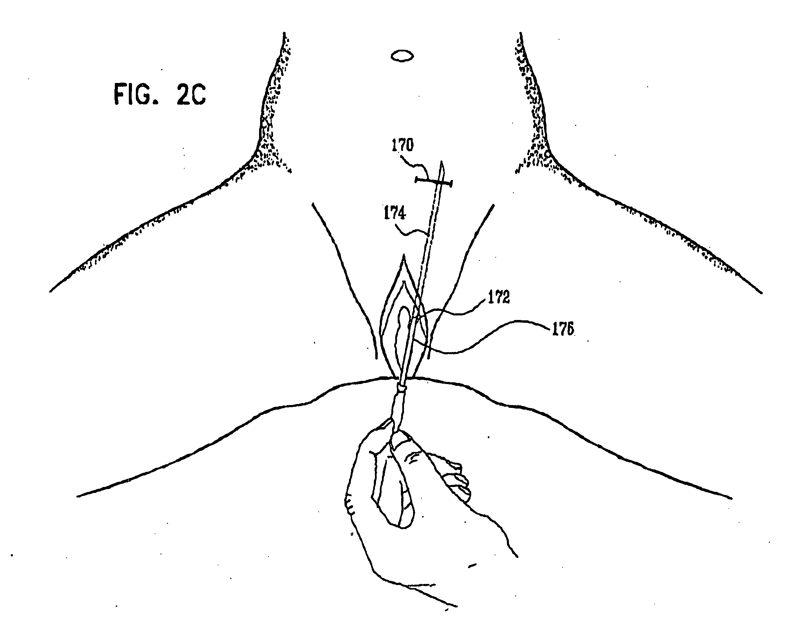

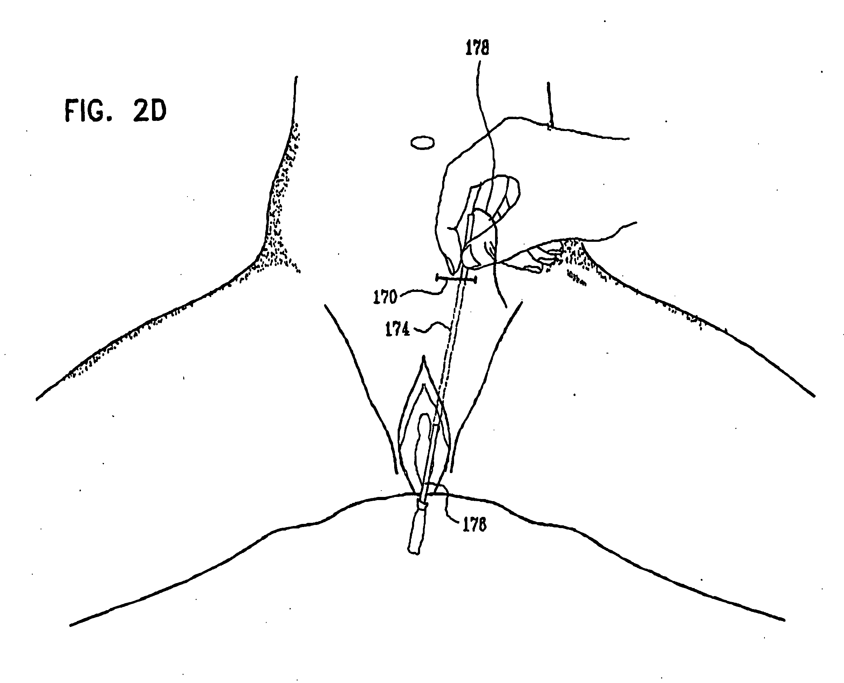

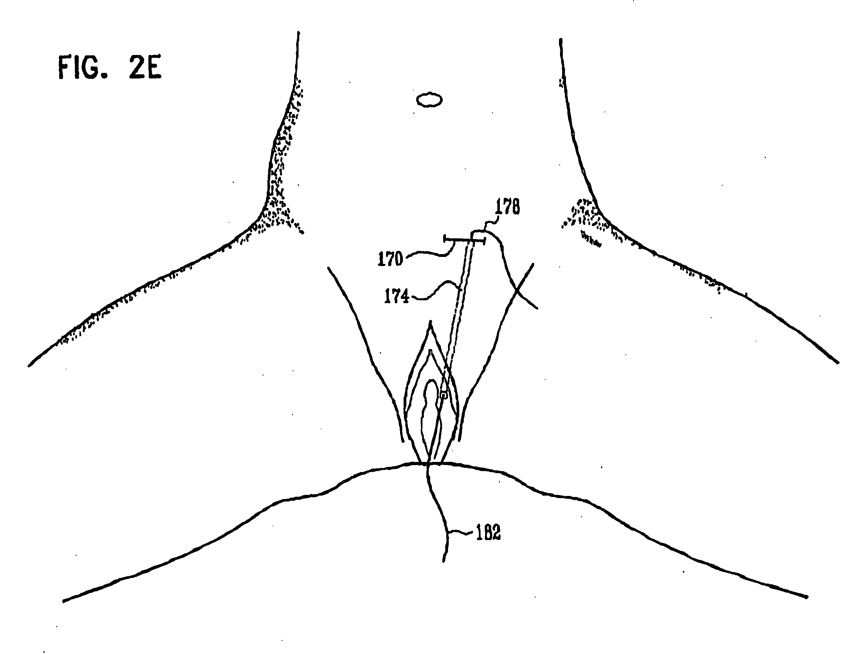

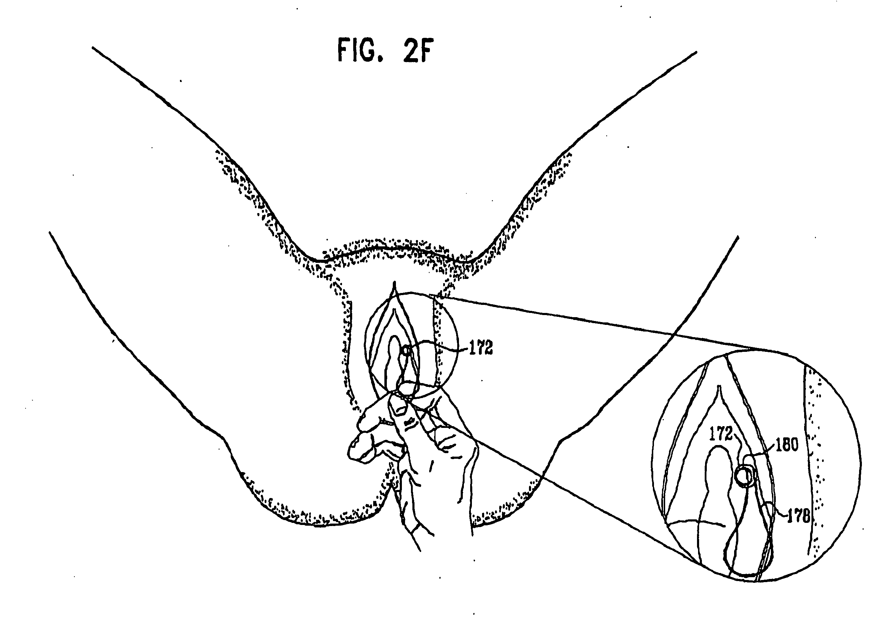

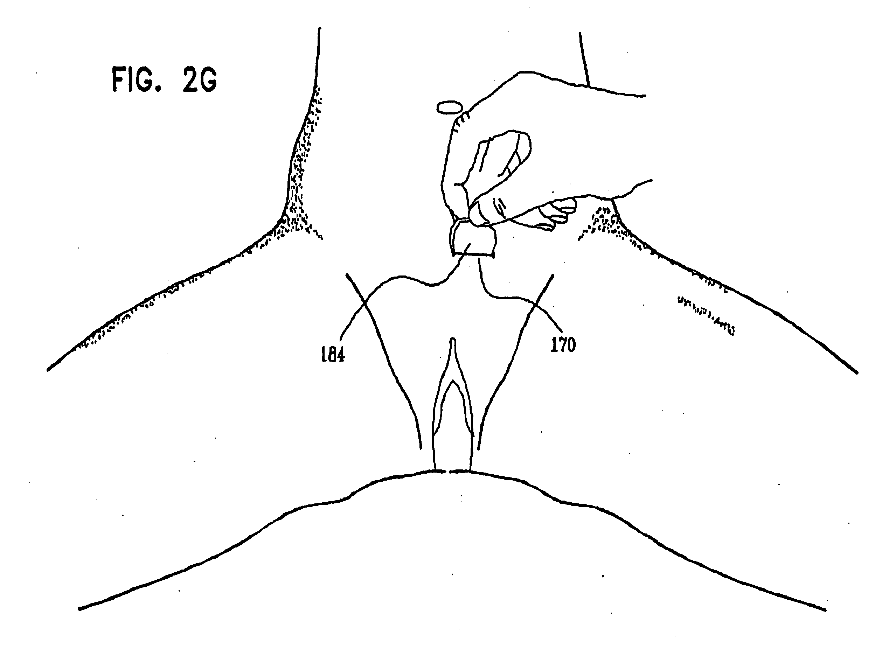

Various aspects of the prior art are described in this section (I) and in greater detail in the following section (II). As described with reference to Figs. 1A and 1B, an electronic stimulator device is preferably implanted in the genital region of a patient suffering from fecal incontinence or urinary incontinence. The implantable medical device generates and delivers electrical stimulation to one or more of the muscles or nerves in the positions or locations described herein, so as to control and treat the patient's incontinence. Preferred methods for implanting the implantable medical device are shown in Figs. 2A, 2B, 2C, 2D, 2E, 2F, and 2G.

Preferably, imminent urge or stress urinary incontinence generates an EMG signal in the muscles that is sensed by one or more electrodes and is analyzed by a control unit of the implantable medical device. Alternatively or additionally, non-EMG signals (e.g., pressure signals) are detected and analyzed by the control unit. When the control unit determines that the signals are indicative of a condition that is likely to cause involuntary urine flow from the bladder, it applies electrical stimulation through the one or more electrodes to pelvic muscles. The electrical stimulation is configured to treat the particular type of incontinence detected (e.g., stress or urge), in order to stimulate a pelvic muscle to contract and inhibit the urine flow.

It is to be understood that although some preferred embodiments are described herein with respect to interpreting EMG signals so as to identify the onset of a particular condition, in many of these embodiments, analysis of pressure signals or other non-EMG signals may be performed instead of or in addition to the analysis of the EMG signals.

For example, the filling of the bowel may be detected by a change in a pressure signal from a pressure sensor that is detected in the operating algorithm of the control unit to apply or alter the application of electrical stimulation to electrodes disposed at particular pelvic muscles as described below in part III.

B. Sensing and control functions of the device

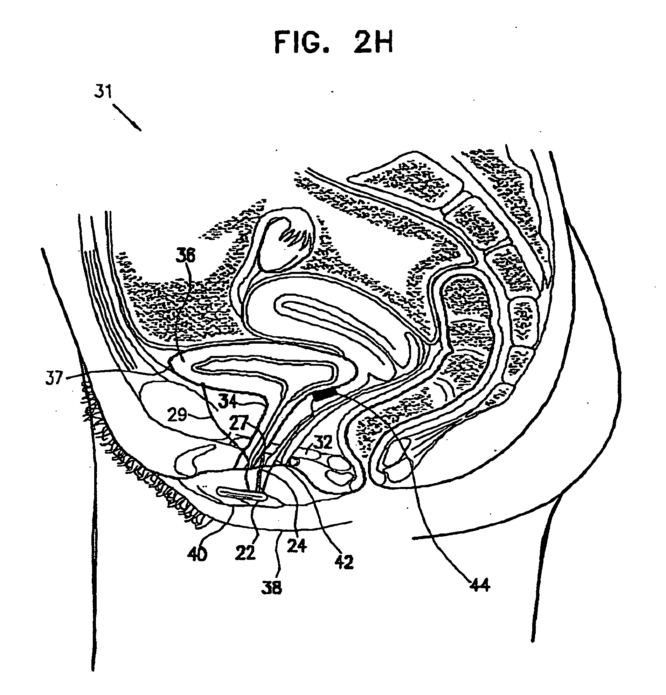

In addition to EMG sensing electrodes, the device preferably also comprises one or more other physiological sensors, described hereinbelow with reference to Figs. 2H, 2I, 3, 4, 10A, and 10B, which generate signals responsive to, for example, motion, intravesical or abdominal pressure, or urine volume in the bladder. These signals are indicative of some forms of incontinence.

Typically, when the urine volume in the bladder is low, there will be no urine flow even when the abdominal pressure does increase. As described with reference to a plurality of the figures, the control unit preferably processes the signals from the various sensors and uses them to determine when the electrical stimulation should be applied to the muscles.

C. Signal processing

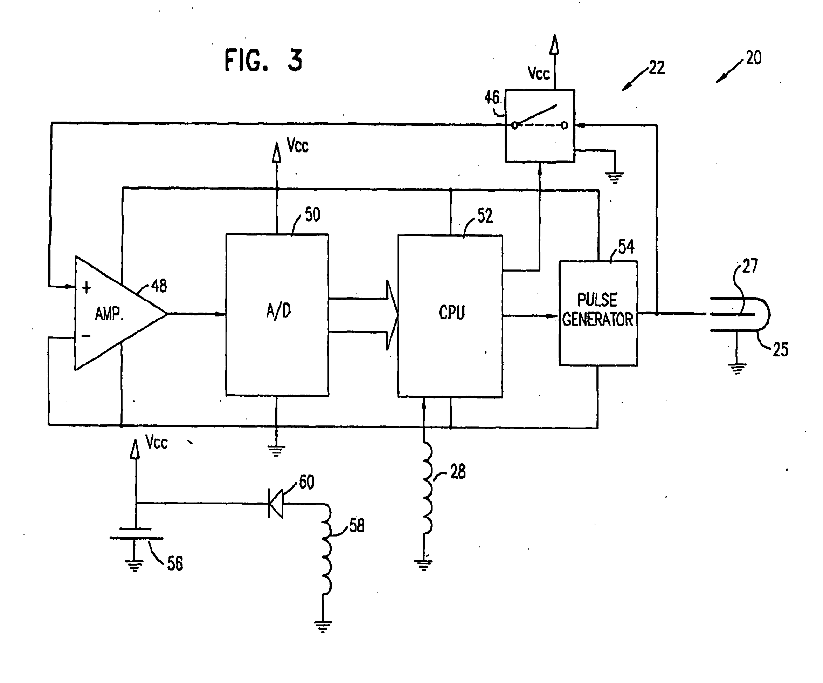

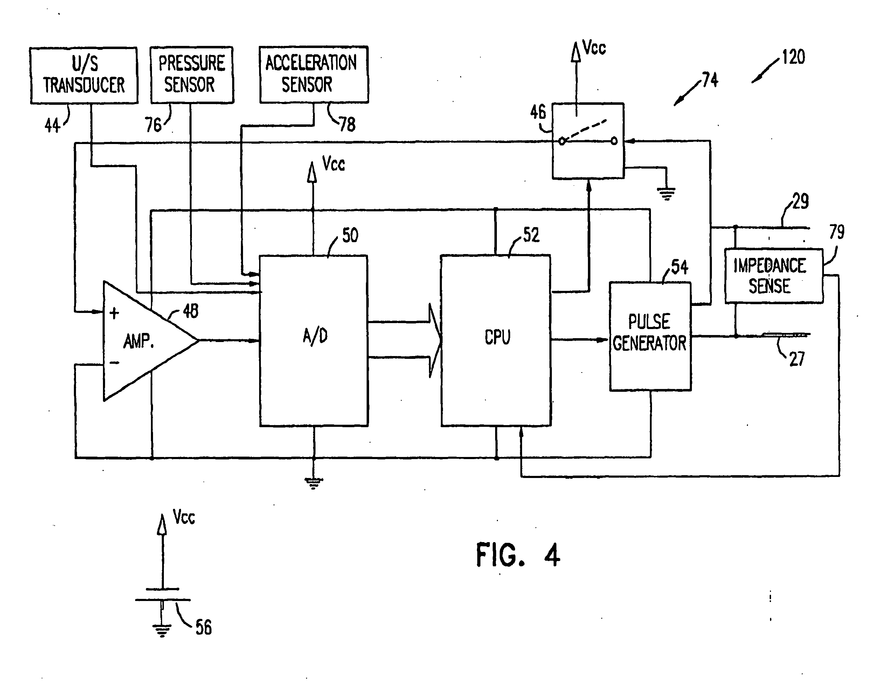

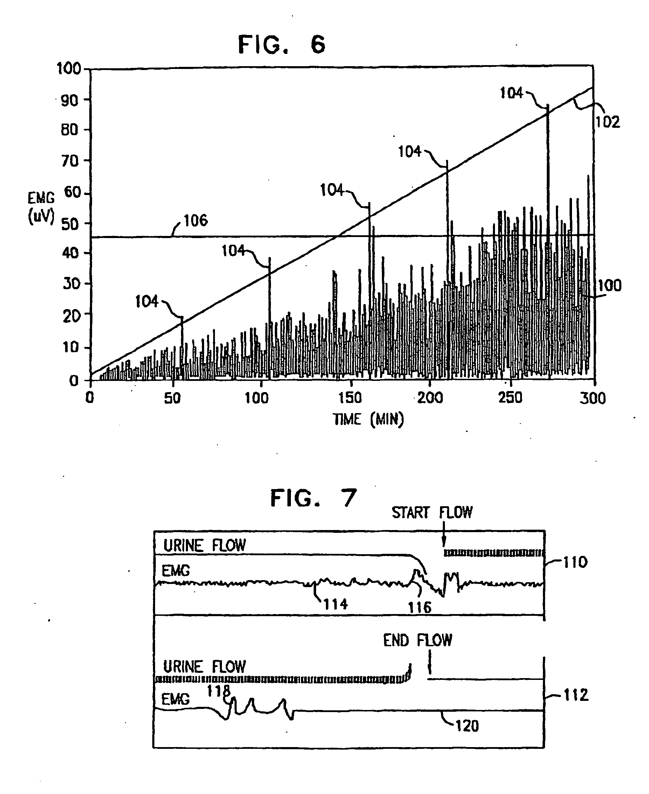

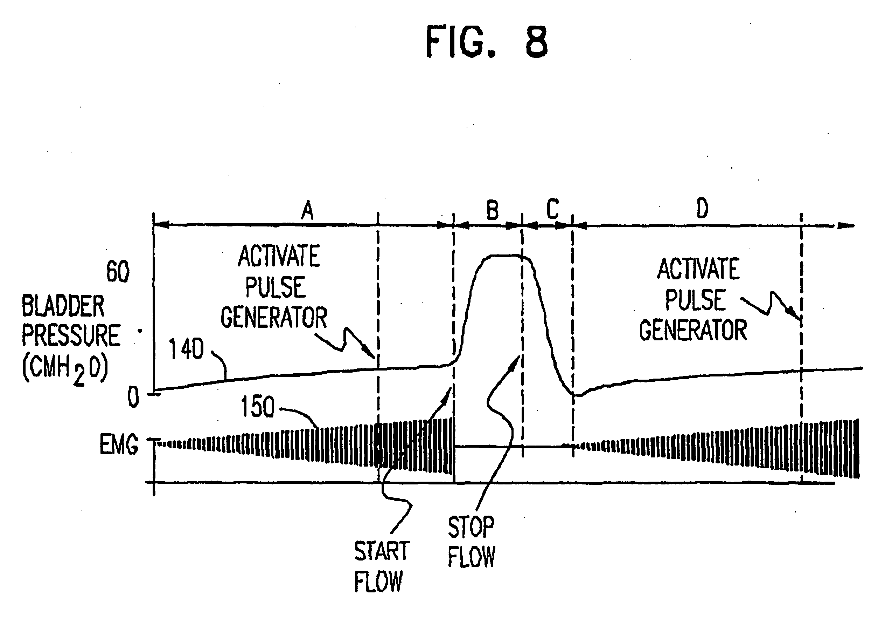

Preferably, the control unit comprises a processor, e.g., as described with reference to Figs. 3 and 4, which is additionally programmed in accordance with an operating algorithm to distinguish between signals indicative of possible incontinence and other signals that do not warrant stimulation of a nerve or muscle. In particular, the processor is preferably programmed to recognize signal patterns indicative of normal voiding, and does not stimulate the muscles when such patterns occur, so that the patient can pass urine normally. Detection of normal voiding is described in more detail with reference to Figs. 7 and 8.

Preferably, the processor analyzes both long-term and short-term variations in the signals, as well as rates, spectral patterns, and patterns of change in the signals. For example, to inhibit stress incontinence, the processor may set a threshold of an aspect of the EMG signal that varies over time responsive to an assessment of the patient's physiological condition. Subsequently, the processor applies the stimulation only when a transient variation in the aspect of the EMG signal exceeds the threshold. Methods for modifying the threshold in real time are described with reference to Fig. 6.

In the context of the present patent application and in the claims, a "time-varying threshold" is to be understood as comprising substantially any appropriate time-varying detection parameters that a person skilled in the art, having read the disclosure of the present patent application, would consider useful in applying the principles of the present invention. By way of illustration and not limitation, these time-varying detection parameters may include magnitude, rate, or other aspects of the EMG signal, or of quantitative ultrasound, pressure, or acceleration measurements, as described herein.

D. Power consumption control

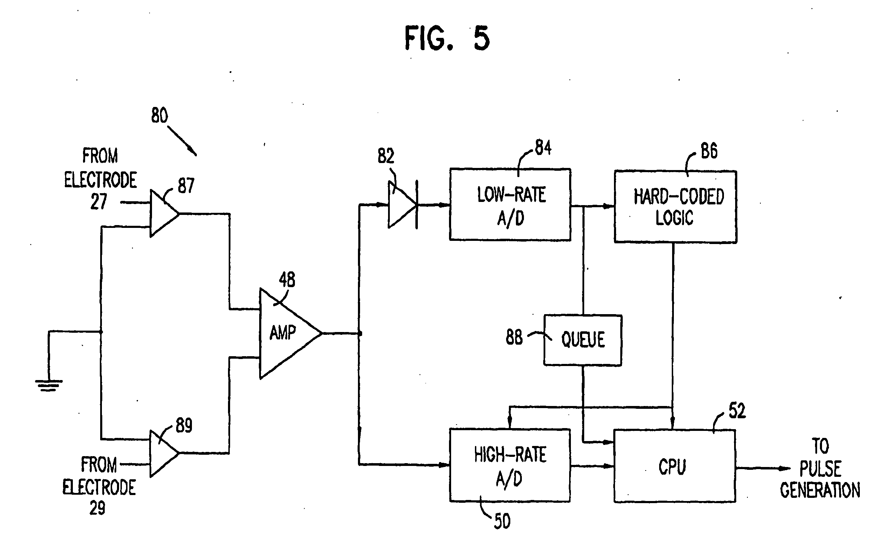

As described with reference to Fig. 5, the control unit preferably comprises a low-power, low-speed processor, which monitors the EMG and/or sensor signals continuously, and a high-speed processor, which turns on only when the low-speed processor detects an increase in EMG or other activity. Use of the two processors has been shown to significantly reduce consumption of electrical power. The high-speed processor performs an accurate analysis of the signals to determine whether stimulation is actually warranted.

Alternatively or additionally, the concepts described herein with respect to two independent processors may be applied using a single processor having two modes of operation - a low power, low capacity mode, and a high power, high capacity mode.

II. Detailed Description of Figures 1 - 10B

A. External elements of a stimulator device

Reference is now made to Fig. 1A, which is a schematic, pictorial illustration of an implantable electronic stimulator device 20. Device 20 is preferably implanted in the pelvic region of a patient, as described further hereinbelow, for use in providing muscle and/or nerve stimulation so as to control and treat urinary urge and stress incontinence.

Device 20 comprises a control unit 22 and electrodes 27 and 29, coupled thereto by medical electrical leads 24. Additionally, device 20 preferably comprises at least one additional physiological sensor 44, such as a miniature ultrasound transducer, one or more accelerometers, a pressure transducer or other sensors known in the art.

The control unit preferably comprises circuitry for sensing electrical signals received by electrodes 27 and 29, such as EMG signals, along with circuitry for processing the signals from sensor 44. Control unit 22 additionally comprises circuitry for applying electrical stimulation to one or both of the electrodes responsive to the signals. Details of control unit 22 and electrodes 27 and 29 are preferably as described in the above-referenced PCT Patent Publications WO 00/19940, entitled "Incontinence Treatment Device," and WO 00/19939, entitled, "Control of urge incontinence," with appropriate changes as described herein or as are otherwise indicated by clinical and engineering considerations that will be clear to those skilled in the art.

The electrodes are preferably flexible intramuscular-type wire electrodes, about 1-5 mm long and 50-100 microns in diameter, thus designed to minimize patient discomfort. They are typically formed in the shape of a spiral or hook, as is known in the art, so that they can be easily and permanently anchored in the muscle. The wire from which the electrodes are made comprises a suitable conductive material, preferably a biocompatible metal such as silver, a platinum/iridium alloy (90/10) or a nickel/chromium alloy. Leads 24 are preferably 5-10 cm long and surrounded by an insulating jacket typically comprising nylon, polyurethane, Teflon or another flexible, biocompatible insulating material. An optional additional wire (not shown) inside the jacket serves as an antenna for the purpose of wireless communications with device 20, as described further hereinbelow.

Control unit 22 preferably comprises circuitry for processing electrical signals received from electrodes 27 and 29 and for generating and applying electrical stimulation to the electrodes. The circuitry is preferably contained in a case made of titanium or other suitable biocompatible metal. Typically, the case is about 20 mm in diameter and 4 mm thick. For some applications, the case serves as a ground electrode for electrodes 27 and 29 when they are sensing or stimulating in a monopolar or unipolar mode. Alternatively, the case may comprise metal coated with a layer of biocompatible plastic, such as polymethyl methacrylate (PMMA) or silicone. Although two electrodes and one sensor are shown attached to the control unit in Fig. 1A, it is possible to use only a single electrode or, alternatively, additional electrodes and/or other sensors, as described further hereinbelow.

Fig. 1 B is a schematic, pictorial illustration of electronic stimulator device 20. Except with respect to the differences described hereinbelow, the embodiment shown in Fig. 1 B is generally similar to the embodiment shown in Fig. 1A, and techniques described herein with respect to one of the configurations can generally be applied to the other configuration, *mutatis mutandis.*

A medical electrical lead 21 is preferably provided to couple control unit 22 to a pelvic muscle of the patient. Lead 21 is secured to the muscle by means of a fixation helix 23 or other techniques known in the art, so as to provide electrical contact between the muscle and two stimulation electrodes 26 and 30 disposed on a silicon casing 19 of the lead. Each electrode is typically less than about 80 mm in length, and is most preferably approximately 3 mm in length. The electrodes are typically separated by approximately 3 mm along the length of lead 21. In this space between electrodes 26 and 30, a tip 15 of an EMG wire 17 may protrude approximately 100 microns through casing 19, for those applications in which EMG sensing is desirable. Typically, the diameter of wire 17 is approximately 50 microns, and the diameter of casing 19 is approximately 1.5 mm.

B. Anatomical and surgical considerations

Figs. 2A, 2B, 2C, 2D, 2E, 2F and 2G show a method for implantation of a pelvic stimulation device. For illustrative purposes, the procedure is shown when performed upon a female patient. Unlike many implantation procedures known in the art, the implantation procedure provided by this example is typically performed under local anesthesia, with the patient placed in the lithotomy position. It will be appreciated that the surgical procedure shown in these figures has further benefits over many similar prior art implantation procedures, in that the complication rate resulting therefrom is significantly reduced by virtue of its being carried out in a region substantially devoid of major blood vessels, and in a manner that avoids risk to delicate structures.

Fig. 2A shows a 4 cm long "pocket" incision 170, made approximately 1 cm cephalad to the pubic bone in order to create a pocket in the subcutaneous tissue adjacent to the fascia. A control unit will later be introduced into this pocket.

Fig. 2B shows a vaginal mucosa incision 172. This second incision, approximately 0.5 - 1 cm long, is preferably made through the vaginal mucosa until the subcutaneous tissue, at a site approximately 0.5 - 1 cm anterior and lateral to the urethral meatus.

Fig. 2C shows the creation of a subcutaneous tunnel 174 using a 12 Fr introducer 176, placed in incision 172, and conveyed subcutaneously until it reaches and exits through incision 170.

Fig. 2D shows the insertion of a stimulation lead 178 through introducer 176 until its exit at the lower end of the introducer.

Fig. 2E shows a stimulation lead tip 182 remaining outside incision 172 after the removal of introducer 176.

Fig. 2F shows the reinsertion of stimulation lead 178 into incision 172. A 5 Fr splittable short introducer 180 is inserted into incision 172, adjacent to lead 178. The introducer is aimed slightly medially, i.e., towards the urethra, care being taken not to injure the urethra. Introducer 180 is pushed for a distance of approximately 2.5 cm, to a site 0.5 - 1 cm lateral to the urethral wall. The free end of stimulation lead 178 is reinserted and advanced through short introducer 180 into the urethral sphincter. Once the stimulation lead is properly secured, introducer 180 is withdrawn by being split into two parts. A 3/0 nylon suture is made in the subcutaneous tissue around the stimulation lead. Subsequently, the free electrode lead is buried subcutaneously, and incision 172 is closed by a 3/0 plain catgut or Dexon suture.

An 8 Fr introducer (not, shown) is inserted through incision 170, between the fascia and muscle tissue, so as to reach the retropubic space. A sensor lead (not shown) for a pressure or electrical sensor is advanced through the introducer to a desired position, e.g., in the retropubic space or between fascia and muscle. Following placement of the lead, it is secured to the fascia by a 3/0 nylon suture. Once the sensor has been properly secured, the lead stylet is withdrawn from the introducer, and the introducer is then removed. Connectors for the sensor lead are connected to appropriate sites on the control unit.

Fig. 2G shows the insertion of a control unit 184 through incision 170. After initial verification of the performance of the implanted system, incision 170 is closed with two layers.

Fig. 2H is a schematic, partly sectional illustration showing the genitourinary anatomy of a female patient 31 in whom device 20 is implanted. It will be understood that, with appropriate changes, device 20 may be implanted in or coupled to a male patient. In this embodiment, electrode 27 is inserted into a muscle 32, such as the levator ani muscle, in a vicinity of urethra 34 and bladder 36. Electrode 29 is inserted into the patient's detrusor muscle 37, which surrounds bladder 36. Alternatively or additionally, electrodes 27 and 29, or additional electrodes not shown in the figure, may be placed in or adjacent to other muscles of the pelvic floor.

The precise placement of the electrodes is typically not essential, particularly since electrical signals tend to pass among the different muscles in the region. The electrodes are preferably inserted through an incision made in the wall of vagina 42. Alternatively, another suitable approach may be chosen for ease of access and minimization of tissue trauma.

Control unit 22 is preferably implanted under the skin in the genitopelvic region of patient 31. Most preferably, the control unit is implanted inside the patient's labia minora 38 or in the labia majora 40. Alternatively, the control unit is not implanted in the patient's body, but is instead maintained outside the body, connected by leads 24 to the electrodes. This configuration is convenient particularly for an initial test period, during which the effectiveness of device 20 in treating a given patient is evaluated before permanent implantation.

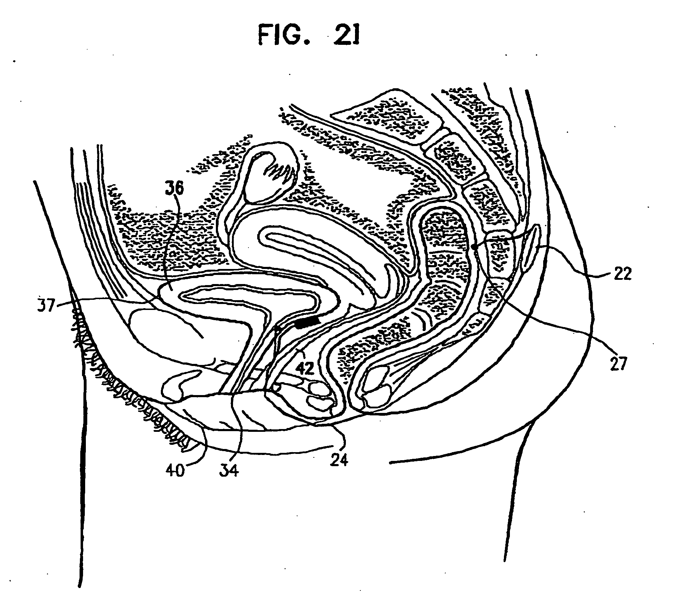

Fig. 2I is a schematic, partly sectional illustration showing the genitourinary anatomy of patient 31 in whom device 20 is implanted. Preferably, control unit 22 is implanted in a vicinity of the sacral spine, as shown, but may alternatively be implanted in the abdomen or in the pelvis. According to this example, the control unit drives electrode 27 to stimulate a nerve that innervates one or more muscles that are responsible for urine control. Typically, a sacral nerve is stimulated, so as to control the flow of urine from the bladder.

Generally, the choice of implantation location for the control unit, as well as which particular nerve is to be stimulated, is made by the patient's physician, responsive to the patient's condition and other surgical considerations. Preferably, electrode 29 (Fig. 2H), is implanted in the detrusor muscle or in another pelvic muscle, and detects EMG signals, which are conveyed for analysis by the control unit. Alternatively or additionally, bladder pressure and volume sensors (not shown) and electrode 29 convey signals to the control unit 22 responsive to bladder contractions associated with imminent incontinence, whereupon the control unit 22: (a) analyzes the signals to distinguish between aspects thereof indicative of stress incontinence and aspects thereof indicative of urge incontinence, and (b) drives electrode 27 to stimulate the sacral nerve and/or drives electrode 29 to stimulate the pelvic muscle, using stimulation parameters appropriate for treating the identified form of urinary incontinence.

C. Signal processing

(i) hardware and algorithms

Fig. 3 is a schematic block diagram showing circuitry used in control unit 22 to receive signals from and apply electrical stimulation to electrode 27. Although in this embodiment device 20 is described as operating in a monopolar (i.e., unipolar) mode, the principles described hereinbelow are applicable to bipolar operation as well, in which both electrodes 27 and 29 are active.

Electrode 27 receives EMG signals from muscle 32, which are conveyed via a normally closed switch 46 to the input of an amplifier 48, preferably a low-noise operational amplifier. Amplified signals output from amplifier 48 are digitized by an analog/digital (A/D) converter 50 and conveyed to a central processing unit (CPU) 52, preferably a microprocessor. Preferably, although not necessarily, the amplified signals are not rectified prior to being digitized, to allow various forms of analysis, for example, spectral analysis, to be performed on the raw data, without the distortion imparted by rectification. CPU 52 preferably analyzes these signals and/or signals from other physiological sensors, such as ultrasound, pressure, strain, and acceleration sensors described hereinbelow, to determine whether they fit a pattern indicating that incontinence is likely to result, and, if so, to determine the type of incontinence. The analysis preferably comprises a spectral analysis and an analysis of EMG signal magnitude and rate. Responsive to a determination that a particular form of incontinence is likely, a pulse generator 54 conveys electrical pulses to electrode 27, as described hereinbelow.

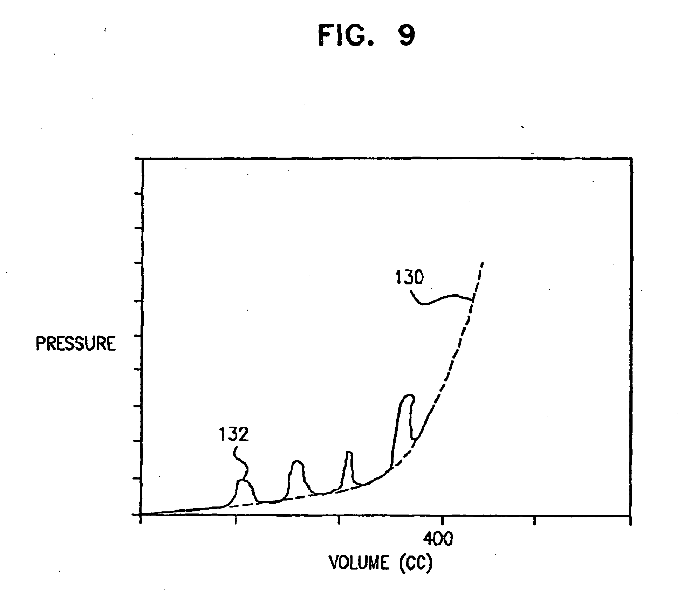

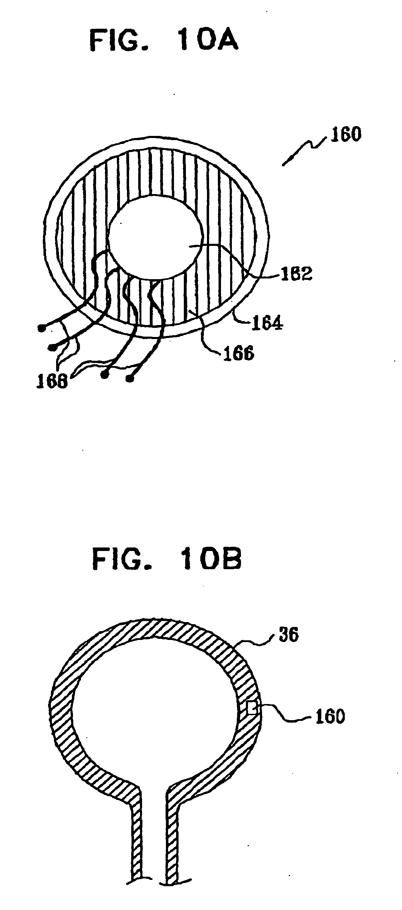

Optionally, sensor 44 (Figs. 1A and 1B) comprises a miniaturized ultrasound transducer, which is implanted in proximity to bladder 36. Additionally or alternatively, sensor 44 comprises a pressure sensor filled with silicon oil, as shown schematically in Fig. 10A. Further alternatively or additionally, sensor 44 comprises a pressure sensor in the bladder, bladder wall, or elsewhere in the abdominal cavity; a strain sensor sutured to the bladder wall; or a sensor that detects action potentials in the bladder muscle. Most preferably, sensor 44 comprises each of these. Signals from the transducer or sensor are conveyed to control unit 22 for analysis, particularly so as to enable the control unit to estimate the urine volume within the bladder. When the bladder is relatively empty, there is no need to actuate electrodes 27 and 29, even when a transient increase in the EMG signal or another signal would otherwise indicate an increased probability of imminent incontinence. Alternatively or additionally, the EMG signal itself may be analyzed to gain an indication of the urine volume in the bladder, since when the bladder is full, the average EMG activity typically increases. Further alternatively or additionally, analysis such as that described hereinbelow with reference to Fig. 9 may be carried out, typically so as to determine the likelihood of imminent urge incontinence.

The CPU is preferably programmed to distinguish between incontinence-related patterns and other signal patterns not associated with incontinence, such as signals generated when patient 31 wishes to pass urine voluntarily. Preferably, the CPU gathers long-term statistical information regarding the EMG and the signals from the other sensors, and analyzes the information to "learn" common signal patterns that are characteristic of patient 31. The learned patterns are used in refining decision criteria used by the CPU in determining whether or not to generate and apply electrical stimulation through the electrodes to tissue. For some applications, a handheld controller (not shown) receives an input from the patient whenever urine is unintentionally passed, and control unit 22 modifies signal analysis parameters and/or stimulation parameters responsive thereto, so as to reduce the likelihood of future incontinence.

(ii) simulation of a typical EMG

Fig. 6 is a graph that schematically illustrates results of a simulation experiment, including a simulated EMG signal 100 of a woman suffering from stress incontinence. A variable, adaptive threshold level 102 is marked on the graph. Over the course of several hours, as the woman's bladder fill level increases, the average level of EMG signal 100 increases accordingly. In this example, threshold level 102 is computed so as to increase as a function of the average EMG. Alternatively or additionally, threshold level 102 and a plurality of other time-varying detection parameters are calculated as functions of other features of the EMG signal or of other aspects of the woman's condition (particularly as measured by sensors 44, 76 and 78 (Fig. 4)), and are used separately or in combination in determining whether to apply stimulation to inhibit involuntary urine flow. As shown, adaptive threshold level 102 enables five possible incidents of incontinence, marked by excursions 104 of signal 100 over level 102, to be detected reliably, with a low false alarm rate. On the other hand, if a fixed threshold level 106 is used, as is known in the art, some EM G excursions 104 are missed (at t = 60 and 110 minutes), and, moreover, the false alarm rate is high (at t > 220 minutes).

(iii) experimentally measured EMG signals: distinguishing incontinence from voluntary voiding

Fig. 7 includes graphs 110 and 112 that schematically illustrate experimental measurements made before, during and after voluntary voiding of urine. Graph 112 is a continuation in time of graph 110. The upper trace in both graphs illustrates urine flow, wherein the beginning and end of voluntary flow are marked by arrows. The lower trace illustrates measured EMG signals.

In a period preceding voiding, an EMG signal 114 shows substantial high-frequency activity, which is generally indicative of a full bladder. Highfrequency spikes in signal 114 (of which none appear in Fig. 7) would be interpreted by CPU 52 as signs of imminent incontinence, leading to actuation of pulse generator 54. On the other hand, voluntary voiding is preceded by a portion 116 of the EMG signal, in which there is a large but gradual increase in the signal level. EMG signal portion 116 is associated with voluntary activation of the pelvic floor muscles for the purpose of passing urine from the bladder, as is a later signal portion 118 during the same act of voiding. Therefore, CPU 52 preferably analyzes not only the level of the EMG signals, but also a rate of change of the signals, in order to distinguish between voluntary and involuntary contractions of the pelvic muscles. When the rate of change is characteristic of voluntary voiding, no stimulation is applied by pulse generator 54.

Fig. 8 (not to scale) includes two graphs, showing: (a) data recorded during a series of periods A, B, C and D, representing stages before, during, and after urination, and (b) preferred times with respect to these periods for activation of pulse generator 54 in order to inhibit urge incontinence. Bladder pressure data 140 and EMG data 150 shown in Fig. 8 are based on text and a figure in the above-referenced book, *Urinary Incontinence* (p. 35), which describes the voluntary voiding of a healthy adult human female subject. Preferably, inputs to control unit 22 include the EMG data and bladder pressure data, to enable the control unit to determine an appropriate time to activate the pulse generator.

During period A, the bladder fills, which filling is preferably detected and identified as such by the control unit. Notably, in period A there is a slow, steady increase in bladder pressure, as well as a slow, steady increase in peak-to-peak amplitude of the EMG signal. Bladder pressure is seen to increase sharply during voiding period B, in comparison to the slow increase of period A. During period C, voiding was terminated. During period D, the bladder fills again, in substantially the same manner as in period A. Examination of periods B and C shows that the EMG signal has essentially zero magnitude during voiding and during its termination, and generally increases with increasing bladder pressure during the bladder-filling periods A and D.

Preferably, control unit 22 identifies an initiation time of normal voiding by analysis of the EMG and/or bladder pressure data. In a preferred embodiment, the control unit actuates pulse generator 54 to apply pulses to electrodes 27 and/or 29 at a predetermined time after voiding. For example, in an interview conducted during the calibration period, it may be determined that a particular patient generally only experiences urge incontinence greater than 1.5 hours following voluntary voiding. The control unit may then be programmed to detect voiding and initiate pulse application one hour thereafter, and to continue the pulse application until a subsequent onset of voluntary voiding is detected.

Alternatively or additionally, the pulse generator may be actuated by the control unit when the average magnitude of the EMG exceeds a specified threshold, because the likelihood of urge incontinence reflects the increased bladder pressure indicated by the EMG signal exceeding the threshold. Further alternatively or additionally, the calibration period may include a training period, in which the control unit continually samples the EMG signal, and in which the patient indicates to the control unit whenever urge incontinence occurs. During or subsequent to the training period, the control unit or an external processor (not shown) analyzes each instance of urge incontinence to determine aspects of the EMG and/or other sensor signals preceding the incontinence which can be used during regular operation of the unit to predict incontinence. For many applications of the present invention, the control unit is operative to execute some or all of the above methods, so as to minimize or eliminate occurrences of urge incontinence. It will be appreciated that these strategies may be applied to other types of incontinence as well, *mutatis mutandis.*

Fig. 9 is a graph showing simulated data, for use in detecting the imminent onset of urge incontinence. Preferably, control unit 22 analyzes a measured pressure-volume (or pressure-time) relationship of the patient's bladder, so as to determine whether the pressure is increasing in a healthy manner, as represented by dashed line 130, or whether it is characterized by one or more relatively sharp features 132, which may indicate detrusor instability and imminent urge incontinence. Preferably, if urge incontinence is deemed likely, then control unit 22 initiates the stimulation of a pelvic muscle using protocols appropriate for treating the urge incontinence (described hereinbelow), which are typically different from those suitable for the treatment of stress incontinence. Measurement of bladder volume may be performed using ultrasound techniques or by means of a strain gauge fixed to the patient's bladder. It is to be understood that whereas a pressure-volume curve is shown in Fig. 9, a pressure-time curve may similarly be generated and subsequently interpreted to identify analogous sharp features indicative of imminent urge incontinence.

Alternatively or additionally, the patient is enabled to instruct control unit 22 to initiate electrical stimulation of the muscles in order to inhibit urge incontinence that the patient senses may be imminent. For example, the patient may input the instruction to the control unit by voluntarily tightening her abdominal muscles, which in turn causes measurable increases in abdominal pressure. Advantageously, the rate of increase of abdominal pressure generated by voluntary contraction of the abdominal musculature is significantly smaller than that increase generated involuntarily, for example, during laughter. Typically, the patient can be taught in a single training session to generate a detectable and distinguishable muscle contraction, appropriate for controlling device 20. For some applications, control unit 22 comprises an external input unit, such as a keypad with buttons designated for certain functions, e.g., "Inhibit urge incontinence now," or "Inhibit stress incontinence now."

In a preferred embodiment, stress incontinence and urge incontinence are distinguished solely (or at least in part) responsive to differences in d(Pressure)/dt characteristic of the respective conditions. For example, values of dP/dt greater than a threshold value are interpreted as being indicative of stress incontinence, while values of dP/dt less than the threshold are interpreted as being indicative of urge incontinence.

D. Muscle stimulation

With reference to Fig. 3, when possible stress incontinence is detected, CPU 52 opens switch 46 and drives pulse generator 54 to apply suitable electrical stimulation to electrode 27 so as to stimulate muscle 32 to contract and thereby inhibit the incontinence that was detected. Switch 46 is opened in order to avoid feedback of the stimulation to amplifier 48, and is closed again after the stimulation is terminated. In the embodiment shown in Fig. 3, the electrical stimulation is applied to the electrode in a monopolar mode, whereby a case 25 of control unit 22 serves as the return (ground) electrode. (This mode can be used only when case 25 comprises a conductive material. When control unit 22 has a non-conductive case, at least two electrodes on one or more leads are generally needed, in order to administer bipolar stimulation.)

For some applications, as muscle 32 contracts, it closes off urethra 34, thus inhibiting the undesired urine flow. Preferably, the electrical stimulation is terminated and switch 46 is closed after a predetermined period of time has passed, e.g., 0.5 - 1 second to treat stress incontinence and 10 minutes to treat urge incontinence. Alternatively or additionally, the electrical stimulation is terminated and switch 46 is closed if the patient voids voluntarily or other new data indicate that the expected incontinence is no longer likely. If possible incontinence is again detected at this point, the electrical stimulation is re-applied.

It will be appreciated that, depending on the particular application, one or more electrical stimulation waveforms may be employed. For example, the electrical stimulation waveform may be monophasic or biphasic and may have a range of amplitudes, duty cycles and/or frequencies. It has been found generally that pulse frequencies in the range between 2 and 50 Hz are effective in engendering contraction of the levator ani and other pelvic muscles, but for some applications it may be appropriate to use frequencies outside of this range. Certain preferred stimulation parameters are described hereinbelow. It has been found generally that duty cycles of about 2 to about 10 seconds on, and about 10 to about 30 seconds off (i.e., about 6% to about 50%) are effective for treating conditions disclosed herein.

Preferably, but not necessarily, the same electrode or electrodes are used to treat both stress incontinence and urge incontinence; however, different stimulation parameters are utilized depending on the particular form of incontinence which is immediately to be treated. Alternatively, at least one electrode is dedicated to treating a particular form of incontinence, e.g., an electrode implanted so as to stimulate the sacral nerve may be driven by control unit 22 to apply current most suitable for treating urge incontinence.

As described hereinabove, the processor preferably identifies the form of incontinence based on particular physiological characteristics detected by the sensors, and control unit 22 applies an appropriate stimulation signal responsive thereto. For example, stress incontinence may be detected using techniques described hereinabove with reference to Figs. 6 and 7, and urge incontinence may be detected using techniques described with reference to Figs. 8 and 9. In patients with mixed incontinence, these techniques are typically sufficient to reveal the significant differences between the two types of incontinence, e.g., the impulsive pressure and/or EMG spikes in instances of stress incontinence are generally not present in urge incontinence, while the pressure-volume and pressure-time features characteristic of detrusor instability and urge incontinence are correspondingly not characteristic of stress incontinence.

For some applications, two sensors are implanted at different sites within the patient that generate signals that are analyzed in combination by control unit 22 so as to determine whether a stress incontinence event or an urge incontinence event is imminent. In a preferred configuration, one pressure sensor is coupled to measure intravesical pressure, while another pressure sensor is coupled to measure intra-abdominal pressure. Sharp increases in bladder pressure that occur generally simultaneously with sharp increases in overall abdominal pressure are typically interpreted to be indicative of possible imminent stress incontinence, e.g., due to laughter. By contrast, increases in bladder pressure that are not accompanied by increases in overall abdominal pressure are interpreted as being indicative of imminent urge incontinence.

Responsive to a determination of imminent incontinence, and the identification of the particular type of incontinence, the stimulation waveform is preferably applied, typically comprising a bipolar square wave having characteristics summarized in Table I. This table also indicates appropriate stimulation parameters for the treatment of other disorders, such as fecal incontinence, interstitial cystitis (IC), chronic pelvic pain, and urine retention, described hereinbelow. For some applications and some patients, other parameters may also be used.

**TABLE I**

| | Stress and fecal incon. | Urge event | Chronic pelvic pain and IC | Urine retention |
|---|---|---|---|---|
| Amp. | 3 - 9V | 0.5 - 5 V | 1 - 4V | 3 - 9V |
| Freq. | 40 - 50 Hz | 5 - 15 Hz | 5 - 15 Hz | 1 - 10 Hz |
| Pulse width | 0.05 - 1 ms | 0.05 - 1 ms | 0.05 - 0.2 ms | 0.05 - 0.2 ms |
| Duration of signal | 0.2-1 s (stress); | 5 - 10 min | 10 - 30min | 20 - 45 s |
| | 1 - 20 s (fecal incon.) | | | |
| Rise time to peak amp. | ~0 | 0 - 1 min | 0 - 3 min | 0 - 5s |
| Decay time | ~0 | 0 - 1 min | 0 - 3 min | 0 - 5 s |
| Optional bursts (Duty cycle) | Bursts not used. | 1-5 s on, 20-60 s off. Typical duty cycle: 5-15% | 2 s on, 20 s off. Typical duty cycle: 5-15% | 2-10 s on, 10-30 s off. Typical duty cycle: 6 -50% |

Thus, it is seen that in response to a determination of imminent stress incontinence, e.g., due to the patient sneezing, high-power electrical stimulation is applied, typically having both a high amplitude and a high frequency. This form of stimulation is generally preferred in inhibiting the rapid onset of stress incontinence, as the stimulation develops significant muscular contraction over a very short time period, so as to prevent the involuntary passing of urine. Shortly after the triggering event (e.g., the sneeze) has finished, the stimulation is preferably removed, because the likelihood of imminent incontinence is diminished.

By contrast, imminent urge incontinence is typically more suitably treated over a longer time period. For example, a signal may be applied from the time that control unit 22 determines that urge incontinence is imminent until the control unit determines that the patient has voluntarily voided. Because of the nature of urge incontinence, i.e., it is characterized by the involuntary and undesired contraction of bladder muscles, lower energy electrical stimulation is applied to a spinal site and/or to a pelvic floor muscle. This lower energy electrical stimulation is preferably configured to induce a relaxation response of the muscle tissue of the bladder, and to thereby inhibit involuntary urination. Advantageously, since the treatment of urge incontinence typically does not consume electrical power at the same rate as the treatment of stress incontinence, the drain on implanted batteries resulting from the treatment of urge incontinence is typically low, allowing the appropriate electrical stimulation to be applied for significantly longer time periods than those useful for treating stress incontinence.

For some applications, the electrical stimulation for treating urge incontinence is applied in bursts, e.g., the electrical stimulation is applied for about 1-5 seconds, and then removed for about 20 - 60 seconds. Typically, the relatively short bursts are sufficient to provide the patient with protection against incontinence during the inter-burst periods. Advantageously, such a protocol of electrical stimulation in bursts further reduces the consumption of electricity.

For example, when treating patients with severe urge incontinence, it is beneficial to treat the urge incontinence prophylactically, i.e., more frequently than when a particular event of urge incontinence is imminent. In this example, electrical stimulation is typically applied automatically, at a fixed time after voluntary voiding and/or whenever bladder volume or pressure exceeds a threshold. Alternatively or additionally, for some patients, the treatment for urge incontinence is applied substantially continuously. Preferably, but not necessarily, these continuous or very-frequent modes of treatment are applied in bursts, as described hereinabove.

For some urge incontinence treatment applications, it is beneficial to extend the initiation of the application of the electrical stimulation over a period ranging from several seconds to about one minute. Thus, for example, a 10 Hz square wave may be increased to a designated waveform application voltage of 2 V over a period of 2 seconds, which is generally fast enough to inhibit urge incontinence, without inadvertently providing a sharp stimulus that might elicit unintentional voiding. When it is desired to apply the electrical stimulation in intermittent bursts, the amplitude is typically held at the peak value for approximately 1-5 seconds, and subsequently caused to decay over a period of several seconds. An extended decay time is also believed by the inventors to inhibit inadvertently eliciting the sharp bladder contractions, which in some instances may bring about incontinence.

Although control unit **22** is generally described herein as distinguishing between stress incontinence and urge incontinence, and applying an appropriate treatment responsive thereto, it is to be understood that other disorders may also be treated some of the techniques described herein, *mutatis mutandis.* Thus, for example, chronic pelvic pain and interstitial cystitis are preferably treated using stimulation parameters shown in Table I. As in the treatment of stress or urge incontinence, the patient herself is typically enabled to activate control unit 22 to treat the condition. Alternatively or additionally, the control unit is programmed to apply appropriate electrical stimulation responsive to a determination of bladder volume (e.g., via an ultrasound measurement), bladder pressure, and/or based on the time from last voiding. Voiding is preferably determined using techniques described herein, such as measuring changes in abdominal pressure, or analyzing pelvic floor EMG data. Typically, interstitial cystitis and chronic pelvic pain are treated, like urge incontinence, using electric signal application parameters configured to induce relaxation of the bladder.

As shown in Table I, pathological retention of urine (a condition common in patients with paraplegia) is preferably treated by the application of electrical stimulation to a pelvic floor muscle having a waveform configured to facilitate voiding. Preferably, the patient is enabled to enter a command into an external controller whenever voiding is desired.

Fecal incontinence can be treated by the application of electrical stimulation to a pelvic floor site or to a site in or adjacent to the anal sphincter of the patient as described in greater detail hereinbelow. Typically, electrical stimulation parameters are generally similar to those for treating stress incontinence. Additionally, because fecal incontinence often accompanies urinary incontinence, particularly stress incontinence, the same techniques described herein for detecting the onset of stress incontinence (e.g., EMG or pressure measurements) are preferably adapted for use in detecting the onset of fecal incontinence.

In normal physiological functioning, an accumulation of feces in the rectum causes afferent signaling that leads to involuntary smooth muscle contraction in the pelvic region and to voluntary contraction of the striated muscle of the anal sphincter. These contractions of smooth and striated muscle provide the control required to defer defecation until a desired time. For some patients, fecal incontinence is caused at least in part by an impairment of the afferent signaling, which should occur responsive to an accumulation of feces.

Therefore, control unit 22 is adapted to enhance the functioning of this afferent pathway, in order to restore normal levels of smooth and/or striated muscle contractions, and, consequently, to restore fecal continence. Preferably, control unit 22 senses the pressure in the patient's rectum, or senses another parameter indicative of rectal filling, and drives electrodes implanted in or near the patient's anal sphincter to apply a signal which generates (or amplifies) afferent signaling. Typically, this induced afferent signaling is sufficient to alert the patient to the gradually increasing level of rectal filling, such that the patient will naturally respond by tightening the striated muscle of the anal sphincter. Often, the induced sensation is indistinguishable from analogous natural sensations experienced by healthy individuals.

Advantageously, smooth muscle contractions are also believed to occur responsive to the induced afferent signaling, such that after a period of weeks to several months, smooth muscle contractions are expected to supplement the striated muscle contractions, and provide enhanced protection against fecal incontinence.

For some applications, the magnitude, frequency, and/or duty cycle of the applied signal is configured to simulate the body's natural afferent signaling patterns, i.e., to have lower values when the rectum is only slightly full, and to increase in value responsive to indications of increased rectal filling.

It is to be appreciated that preferred stimulation parameters are described herein by way of illustration and not limitation, and that electrical stimulation waveforms comprising, for example, biphasic and/or monophasic components, a decaying square wave, a sinusoid or sawtooth waveform, or any other shape known in the art to be suitable for stimulating muscle or nervous tissue can also be used. Generally, appropriate waveforms and parameters thereof are determined during an initial test period of device 20, and are updated intermittently, either in a healthcare facility or automatically during regular use.

E. Provision of power to the control unit

With reference to Figs. 3 and 4, power is supplied to the elements of control unit 22 by a battery 56, which may comprise a primary battery (non-rechargeable) and/or a rechargeable battery. Alternatively, a super-capacitor, as is known in the art, may be used to store and provide the electrical power. If a rechargeable battery or super-capacitor is used, it is preferably recharged via an inductive coil 58 or antenna, which receives energy by magnetic induction from an external magnetic field charging source (not shown) held in proximity to the pelvis of patient 31. The magnetic field causes a current to flow in coil 58, which is rectified by a rectifier 60 and furnished to charge battery 56. An optional coil 28, coupled to CPU 52 for the purpose of wireless communications with device 20, may also be used for charging the battery.

Preferably, battery 56 comprises a standard battery, such as a lithium battery, having a nominal output of 3 volts. Most preferably, pulse generator 54 comprises a DC/DC converter, as is known in the art, and a capacitor, which is charged by the DC/DC converter to a constant, stepped-up voltage level regardless of the precise battery voltage, which may vary between 3.5 and 1.8 volts. The same DC/DC converter, or another similar device, preferably supplies power to other circuit components of control unit 22.

F. External communication with the control unit

An inductive arrangement including coil 28 is preferably used to program the CPU, using an external programming device (not shown) with a suitable antenna. Alternatively, the programming device generates a modulated magnetic field to communicate with a receiver inside case 25 that preferably senses the field using a Hall effect transducer. Such programming may be used, for example, to set an amplitude or duration of the stimulation waveform applied by pulse generator 54, or to set a threshold level or other parameters, according to which the CPU distinguishes between EMG signals or other signals that are indicative of impending urge or stress incontinence and those that are not (e.g., those that indicate voluntary voiding). Such programming may be carried out by medical personnel or by the patient herself, who can similarly turn the implanted control unit on and off as desired by passing a suitable magnet over her pelvis.

Although the circuit blocks in control unit 22 are shown as discrete elements, some or all of these blocks are preferably embodied in a custom or semi-custom integrated circuit device, as is known in the art.

G. Utilization of other sensors

Fig. 4 is a schematic block diagram illustrating a muscle stimulator device 120. Device 120 is substantially similar to device 20, except for features described hereinbelow. Device 120 comprises a control unit 74, which is coupled to electrodes 27 and 29. Electrode 29 also serves as a sensing electrode that conducts EMG signals via switch 46 to amplifier 48, as described hereinabove. Alternatively, electrodes 27 and 29 may be coupled as differential inputs to amplifier 48. Pulse generator 54 applies the stimulation between electrodes 27 and 29 in a bipolar mode.

In addition to or instead of the EMG signals received from electrode 29, CPU 52 preferably receives additional signals from other physiological sensors, such as an ultrasound transducer, a pressure sensor 76 and/or an acceleration sensor 78, or other types of strain and motion measurement devices, as are known in the art. Pressure sensor 76 is preferably implanted on or in bladder 36, so as to detect increases in abdominal or intravesical pressure that may lead to involuntary urine loss. Similarly, acceleration sensor 78 is preferably implanted so as to detect bladder motion associated with hypermobility, which is similarly associated with urine loss. The additional signals from these sensors are preferably analyzed by the CPU together with the EMG signals in order to improve the accuracy and reliability of detection of impending incontinence.

An impedance sensor 79 is preferably used to measure the tissue impedance between leads 27 and 29, using physiological impedance measurement techniques known in the art. During long-term use of device 120 (or other such devices), fibrosis in the area of the implanted electrodes tends to cause the impedance to increase, so that the stimulating current for a given applied voltage decreases. The impedance measured by sensor 79 is used as a feedback signal instructing CPU 52 to increase the voltage, so that a generally constant level of stimulation current is maintained.

Fig. 10A is a schematic illustration (not to scale) showing details of a sensor 160 for measuring intravesical pressure. Sensor 160 preferably comprises a pressure-sensitive element such as a piezoelectric element or a piezoresistive element 162. Element 162 is typically surrounded by silicon oil 166 or a similar liquid, which, in turn, is contained within a flexible wall 164. Preferably, element 162 is connected by four leads 168 to control unit 22. Leads 168 are preferably coupled in a Wheatstone bridge formation, such that pressure on wall 164 induces a change in resistance of piezoresistive element 162 that, in turn, is detected by control unit 22. Typically, control unit 22 applies a voltage across two of the leads, and senses and amplifies the voltage developed across the other two leads in order to ascertain the pressure being applied to sensor 160. In order to increase battery life, the voltage applied across the leads is preferably applied in short pulses (e.g., 50 microseconds on, 30 milliseconds off).

Fig. 10B is a schematic illustration (not to scale) showing sensor 160 implanted in the muscle wall of bladder 36. Typically, one or more sensors 160 are implanted in or on the bladder wall or elsewhere in the abdominal cavity.

H. Reduction of power consumption

Fig. 5 is a schematic block diagram showing details of signal processing circuitry 80 for use in device 20 or 120. In order to detect impending incontinence with adequate reliability, A/D converter 50 optimally samples the EMG signals from the electrodes at 1000-5000 Hz, and CPU 52 preferably performs a detailed analysis of the sample stream. Systems for incontinence control known in the art, operating at sample rates below 1000 Hz, cannot adequately distinguish between signals that may be indicative of incontinence and those that are not. For the purpose of such high-rate sampling, CPU 52 preferably comprises a low-power, software-programmable processor. If A/D converter 50 and CPU 52 were to operate continuously, however, battery 56 would rapidly run down. Therefore, circuitry 80 comprises a low-power, low-resolution A/D converter 84 and hard-coded processing logic 86, which operate continuously at a low sampling rate, preferably at about 100-200 Hz. Input from amplifier 48 to A/D converter 84 is preferably rectified by a rectifier 82.

In operation, A/D converter 50 and CPU 52 are normally maintained in a standby state, in which their power consumption is negligible. When logic 86, operating at the low sampling rate, detects EMG signals that may be a precursor to incontinence, it signals A/D converter 50 to begin sampling at the high rate. In order not to lose significant data from the brief period before A/D converter 50 and CPU 52 turn on, signals from A/D converter 84 are preferably stored in a cyclic (or first-in first-out) queue 88, such as a delay line. The entire sequence of signal detection and processing is estimated to take between 5 and 20 ms, up to the point at which CPU 52 reaches a decision as to whether or not to actuate pulse generator 54. Pulse generation takes between 1 and 20 ms, with the result that contraction of the pelvic muscles begins within 15-50 ms of an onset of increased EMG activity indicating impending urine loss. Thus, urethra 34 is substantially closed off before any significant amount of urine can leak out.

As shown in Fig. 5, EMG inputs from electrodes 27 and 29 are preferably amplified before processing in a dual-differential configuration, so as to afford enhanced sensitivity and reduced noise. Electrodes 27 and 29 are coupled to respective differential preamplifiers 87 and 89, the outputs of which are differentially amplified by amplifier 48.

III. Systems and methods for treating fecal incontinence and related pelvic floor disorders

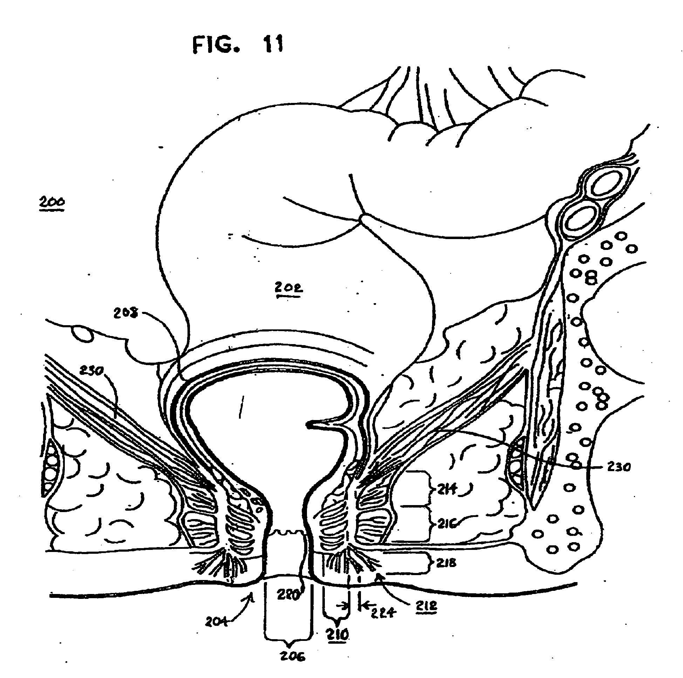

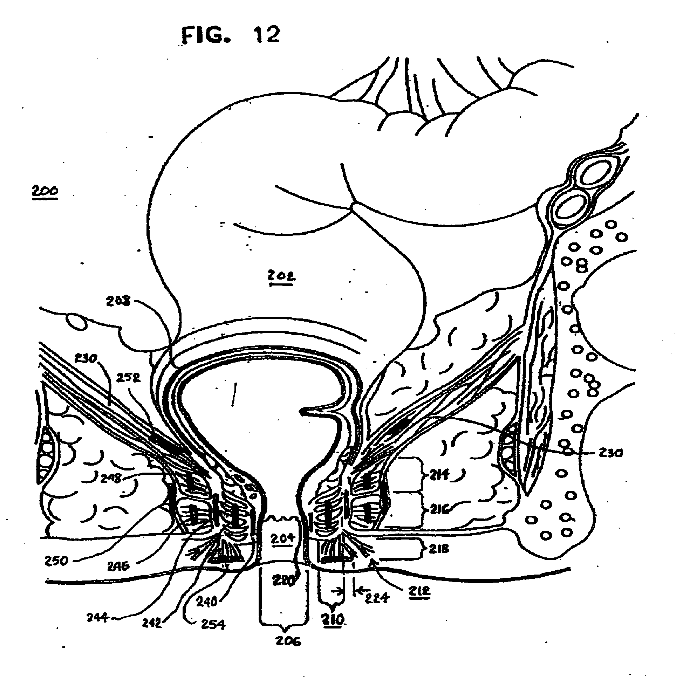

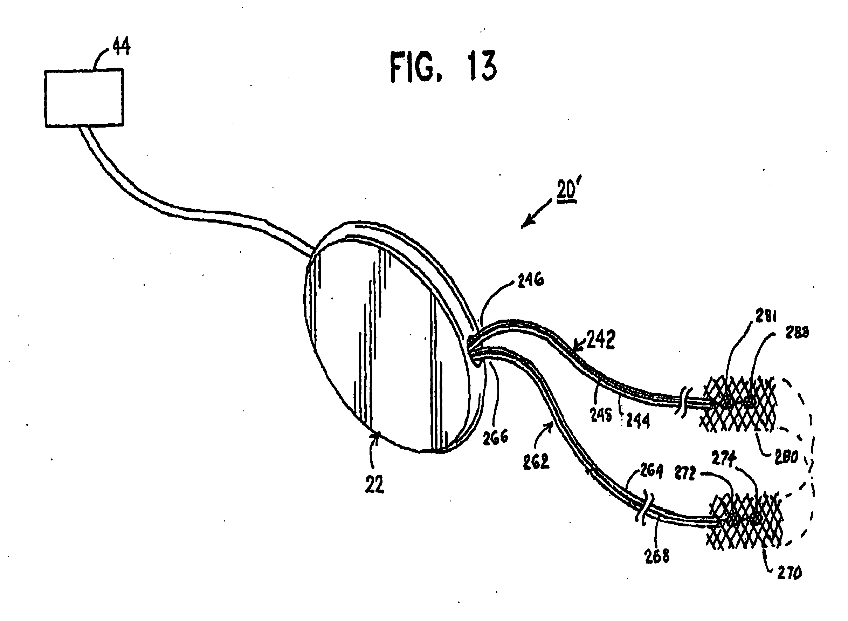

As described hereinabove, the implantable electronic stimulator device 20 comprising the control unit 22, lead(s) 24, and optionally physiological sensor 44 or 160 may advantageously be employed to provide electrical stimulation to the pelvic floor musculature and in particular to one or more of the internal anal sphincter, the external anal sphincter, and the levator ani (as well as the puborectalis muscle) to treat or control fecal incontinence. These muscular structures of the pelvis are first described in reference to Fig. 11, and then particular locations for stimulation electrodes are described in reference to Fig. 12. Particular medical electrical leads for maintaining the electrode position and (in some instances) for supporting the anus or lower part of the rectum are then described in reference to Figs. 13-17.

The muscles of the pelvis can be characterized as forming the pelvic diaphragm or floor and the pelvic wall that support and contain the bladder, rectum, and reproductive organs.

The pelvic floor includes the fascia-covered coccygeus and the levator ani muscles, which function as the pelvic diaphragm, separating pelvic viscera from the perineal structures inferiorly. The pelvic diaphragm counters abdominal pressure and, with the thoracic diaphragm, assists in micturition, defecation, and childbirth. It is an important support mechanism for the uterus, resisting prolapse.

The coccygeous is the posterior muscle of the pelvic floor on the same plane as the iliococcygeous. The levator ani (anal lifting or elevating) on each side arises from the pubic bone and ischial spine and the intervening tendinous arch, droops downward as it passes through the midline, and inserts on the anoococcygeal ligament and the coccyx with the contralateral levator ani. The levator ani essentially has four parts, the levator prostatae/vaginae (male/female), the puborectalis, the pubococcygeus, and the iliococcygeous.

Viewed from above, the levator prostatae/vaginae extends posteriorly from attachment to the pubic bone around the urethra.

The puborectalis extends from attachment to the pubic bone posteriorly around the levator prostatae/vaginae, the urethra, and the rectum.

Right and left branches of the pubococcygeus extends from attachment to each tendinous arch, posteriorly around the puborectalis levator, and along either side of the prostatae/vaginae, the urethra, and the rectum.

Right and left branches of the iliococcygeous extends from attachment to each tendinous arch posteriorly alongside the pubococcygeus, the puborectalis levator, the prostatae/vaginae, the urethra, and the rectum.

Turning to the pelvic wall, it is formed by the obturator intemus and piriformis muscles and the sacrotuberous and sacrospinous ligaments.

The right and left obturator intemus muscles are lateral rotators of the right and left hip joints. The right and left obturator intemus arise, in part, from the margins of the right and left obturator foramens (comprised of both the intemus and externus layers) on the pelvic side, pass downward and posterolaterally past each obturator foramen to and through the lesser sciatic foramen, inserting on the medial surface of the greater trochanter of each femur. The right and left tendinous arches are fascia coverings of the right and left obturator internus muscles.

The right and left piriformis muscles are lateral rotators of the hip joint that each follow a course similar to the right and left obturator intemus to attachment with the greater trochanters of the right and left femurs.

The levator ani supports the lower end of the rectum and bladder during the controlled efforts of expulsion of feces and urine. As it is fixed to the coccyx, it helps to fix the central point of the perineum, so that the bulbocavernosus may act from this fixed point.

The levator ani is always in a state of tonic contraction and keeps the anal canal and orifice closed. The levator ani is also a voluntary or volitional muscle that can be willed to contract more forcefully to more firmly occlude the anal canal in expiratory efforts unconnected with defecation.

Moreover, the contraction force of the levator ani may be increased by direct electrical stimulation of the levator ani muscle fibers. Unless otherwise indicated, it will be understood that references herein to the electrical stimulation of the levator ani embraces the location of stimulation electrodes in or related to any of the four parts.

Turning to Fig. 11, the inferior portion of the gastrointestinal tract 200 includes the rectum 202 that terminates in the anus 204 surrounding the anal canal 206 and anal orifice (dilated for ease of illustration). The anal canal 206 typically extends about 4 to 5 cm superior to the anal orifice. The rectum 202 is formed by a rectal wall 208 substantially centered on a centerline of the rectum. The rectal wall 208 comprises an exposed mucosal layer overlying a submucosal layer that overlies rectal wall muscle layers. The rectal wall muscle layers comprise a circular muscle layer adjacent the submucosal layer and extending around the rectum 202 and a longitudinal muscle layer adjacent the circular muscle layer and extending transversely to the circular muscle layer and substantially parallel to the centerline of the rectum 202.

The anus 204 further includes the anal sphincter within the anal wall surrounding and defining the anal canal 206 and comprising sphincter ani intemus or internal anal sphincter 210 and the sphincter ani externus or the external anal sphincter 212. Generally speaking, the internal anal sphincter 210 surrounds the anal canal 206, and the external anal sphincter 212 surrounds the internal anal sphincter 210 and extends somewhat inferior to the internal anal sphincter 210. A potential space characterized as the intersphincteric space 224 exists between the internal and external and anal sphincters 210 and 212. The fat of the ischio-rectal fossae laterally surrounds the external anal sphincter 212.

The external anal sphincter 212 is a thin flat plane of striated muscle fibers that are always in a state of tonic contraction to keep the anal orifice closed. The closed anal canal 206 therefore has the appearance of a longitudinal slit. The striated muscle fibers of the external anal sphincter 212 are further differentiated as the deep external anal sphincter fibers 214, the superficial external anal sphincter fibers 216, and the subcutaneous external anal sphincter fibers 218. Posteriorly, the fibers are not attached to the coccyx, but continuously extend around the anal canal 206. The superior boundary of the deep external anal sphnicter fibers of the external anal sphincter 212 is ill defined as the muscle fibers merge with the levator ani 220.

The external anal sphincter muscle fibers can be voluntarily or volitionally placed in a greater condition of contraction, to more firmly close the anal orifice 206. Moreover, the contraction force of the external anal sphincter 212 may be increased by direct electrical stimulation of the sphincter muscle fibers. Unless otherwise indicated, it will be understood that references herein to the disposition of stimulation electrodes 27, 29 in or with respect to the external anal sphincter 212 embraces positions related to all of these muscle fibers.

The internal anal sphincter 210 is a ring of smooth muscle fibers that surrounds the lower extremity of the rectum 202 and the anal canal 206. The smooth muscle fibers of the internal anal sphincter 210 are in a constant state of contraction and are incapable of voluntary or volitional control. The internal anal sphincter 210 is contiguous with the inferior terminus of the circular rectal wall muscle layer and is supported by the levator ani 230, particularly the puborectalis part. For purposes of definition, the transition between the circular rectal wall muscle and the internal anal sphincter 210 corresponds to the transition or anorectal border between the rectum 202 and the anus 204. The inferior border of the internal anal sphincter 210 is contiguous with the external anal sphincter 212, particularly the subcutaneous external anal sphincter fibers.

In a continent person, the voluntary external anal sphincter 212 works with the involuntary internal anal sphincter 210 to occlude the anal canal 206. The internal anal sphincter 210 contributes about 85% of the resting tone of occlusion of the anal canal 206, to keep fecal material in the rectum 202 until controlled expulsion is volitionally initiated. The contraction force of the internal anal sphincter 210 may be increased by direct electrical stimulation of the sphincter muscle fibers.

Thus, the anal sphincter of a continent person closes the anal canal 206 and normally prevents involuntary expulsions from the lower bowel and rectum 202. Voluntary defecation is aided by a process of sensing fecal matter and relaxing the internal and external anal muscle fibers as follows.

A pectinate (dentate) line 220 transverse to the rectum axis is defined about 2.5 to 3 cm superior to the anal orifice. The superior extent of the external anal sphincter 212 extends about 5 cm above the pectinate line 220. The superior extent of the internal sphincter muscle extends about 2 to 2.5 cm above the pectinate line.

A band of mucosal tissue immediately superior to the pectinate line 220, referred to as the anal columns, is sensitive to the presence of fecal material. The anal columns provide sensory information that discriminates among different types and textures of fecal material, thereby aiding in overall control of the discharge of fecal material.

Sensitive mucosal tissue, called the anoderm, lines the anal canal 206 inferior to the pectinate line 220. Anoderm tissue is sensitive to contact with fecal material such that the sensed presence of fecal material initiates the release of tension by the anal sphincter to facilitate discharge through the anal canal 206. In a person suffering from fecal incontinence, the external anal sphincter 212 or the internal anal sphincter 210, or both, lose muscle tone, and the anal canal 206 and orifice are not fully constricted by the anal sphincter. Fecal material that therefore passes by the pectinate line spontaneously excites the sensitive anoderm tissue initiating an immediate discharge response, resulting in an incontinent event.

Because of their important sensory functions, treatment of the rectum 220 and anus should guard against damage to the mucosal tissue below and above the pectinate line 220. This sensitive mucosal tissue may be damaged, e.g., by exposure to abnormal heat, and typically do not regenerate after thermal injury.

Turning to FIG. 12, various locations of stimulation electrodes for stimulating one or more of the internal anal sphincter 210, the external anal sphincter 212 and the levator ani 230 are depicted. It will be understood that one or more electrode may be disposed in or in relation to each of the ringshaped internal and external anal sphincters 210 and 212 extending around the anus 204 and/or in or in relation to the levator ani 230 and/or in or in relation to perineal floor muscles.

Thus, a first depicted electrode location or position 240 is adjacent the internal anal sphincter 210 beneath the mucosal tissue lining the anal canal 206. One or more stimulating electrode may be supported on a band or mesh support at the distal end of a medical electrical lead that is implanted to extend around the anal canal 206.

A second depicted electrode location or position 242 is within the internal anal sphincter 210 extending around the anal canal 206. One or more stimulating electrode may be supported on a band or mesh support at the distal end of a medical electrical lead that is implanted to extend through the internal anal sphincter 210 and around the anal canal 206.

A third depicted electrode location or position 244 is within the intersphincteric space 224 between the internal and external and anal sphincters 210 and 212 extending around the anal canal 206. One or more stimulating electrode may be supported on a band or mesh support at the distal end of a medical electrical lead that is implanted to extend through the intersphincteric space 224 and around the anal canal 206.

A fourth depicted electrode location or position 246 is within the superficial external anal sphincter fibers 216 of the external anal sphincter 212 extending around the anal canal 206. One or more stimulating electrode may be supported on a band or mesh support at the distal end of a medical electrical lead that is implanted to extend through the superficial external anal sphincter fibers 216 of the external anal sphincter 212 and around the anal canal 206.

A fifth depicted electrode location or position 248 is within the deep external anal sphincter fibers 214 of the external anal sphincter 212 extending around the anal canal 206. One or more stimulating electrode may be supported on a band or mesh support at the distal end of a medical electrical lead that is implanted to extend through the deep external anal sphincter fibers 214 of the external anal sphincter 212 and around the anal canal 206.

A plurality of stimulation electrodes in electrode locations or positions 246 and 248 may be supported on a band or mesh support at the distal end of a medical electrical lead that is implanted to extend through the deep and superficial external anal sphincter fibers 214 and 216 of the external anal sphincter 212 and around the anal canal 206.

A sixth depicted electrode location or position 250 is adjacent to and surrounding one or both of the deep and superficial external anal sphincter fibers 214 and 216 of the external anal sphincter 212 and around the anal canal 206. One or more stimulating electrode may be supported on a band or mesh support at the distal end of a medical electrical lead that is implanted to extend around the deep and/or superficial external anal sphincter fibers 214 and 216 of the external anal sphincter 212 and around the anal canal 206.

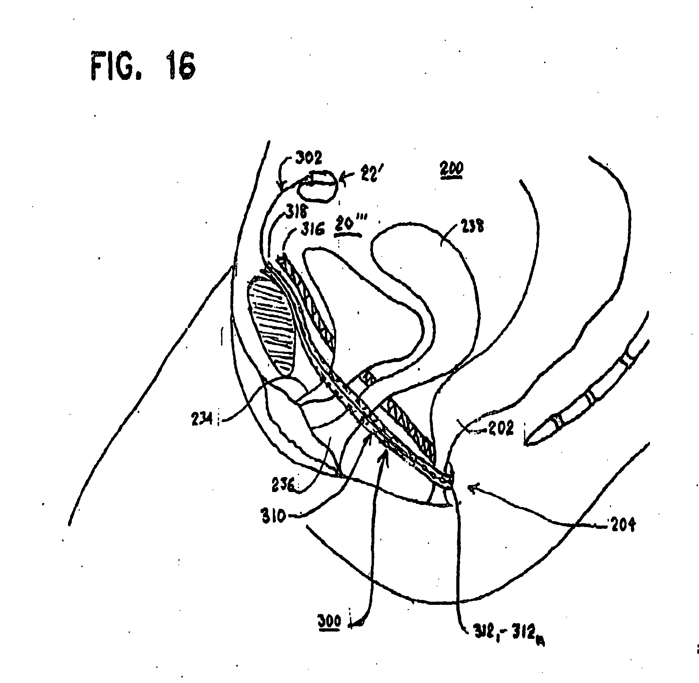

Any of the above-described and depicted electrode locations or positions 240, 242, 244, 246, 248, 250 may comprise a band extending around the anal canal 206 defined by the tissue pathway of a fecal sling as described further below in reference to Fig. 16 that provides mechanical support to the anal sphincter of anus 204. Methods for positioning the sling are described in the above-referenced U.S. Patent Application Publication Nos. 2002/0161382 and 2004/0039453 and a modification of U.S. Patent Nos. 6,911,003 and 6,612,977 (via the transobturator or suprapubic methods, either up to or through the obturator foramen) along with introducers/needles that can be used to place the sling in a desired location. Fecal continence can also be achieved with a mesh or graft being placed at or adjacent the levator ani muscle(s) and thereafter populated with one or more electrodes. The mesh can be introduced through a perineal or tranvaginal approach.

A seventh depicted electrode location or position 252 is on, adjacent to or within the levator ani muscle 230 where it supports the rectum 202 and near the border with the deep external anal sphincter fibers 214 of the external anal sphincter 212. The electrode location or position 252 may comprise a band of the levator ani inferior to the rectum 202 that conforms to the tissue pathway of a fecal sling as described further below in reference to Fig. 16 that provides mechanical support to lower rectum 202 as well as the anal sphincter of anus 204.

An eighth depicted electrode location or position 254 is on, adjacent to or within the subcutaneous pelvic floor muscle fibers that may include or comprise subcutaneous external anal sphincter fibers 218 surrounding the anal orifice to the anal cavity 206 within the perineum. The electrode location or position 252 may comprise a band of the subcutaneous external anal sphincter fibers 218 that conforms to the tissue pathway of a fecal sling as described further below in reference to Fig. 16 that provides mechanical support to the perineum as well as the anal sphincter of anus 204.

It will be understood that the practice of certain methods described herein contemplates locating or positioning stimulation electrodes at more than one of the depicted electrode locations or positions, e.g., at locations 240 and 244 or 240 and 250 to selectively apply stimulation to the internal anal sphincter 210 and the external anal sphincter 212. Alternatively, one or more stimulation electrodes may be placed at locations 252 and other stimulation electrodes may be placed at the other locations, e.g., at location 254 adjacent or within the perineal floor muscle fibers.

Moreover, the medical electrical leads can employ passive electrode stabilization or fixation mechanisms to maintain the stimulation electrodes disposed in the above-described and depicted electrode locations or positions 240, 242, 244, 246, 248, 250, 252, and 254. The preferred passive fixation mechanism comprises a mesh that the stimulation electrode(s) is affixed to. A single stimulation electrode or a plurality of stimulation electrodes may be supported on the mesh and the electrode(s) may be exposed on both sides of the porous mesh or insulation may be applied to the surfaces of the electrodes on one side of the mesh to enable stimulation in only one direction. The stimulation electrodes may also comprise electrically conductive strands of the mesh. Multiple stimulation electrodes may be distributed in a pattern and spacing to ensure distribution in the locations or positions extending around the anal canal 206 or the levator ani 230 or the perineal floor.

For example, in reference to Fig. 13, an implantable electronic stimulator system or device 20' is schematically depicted comprising the above-described control unit 22 and the optional fecal presence sensor 44 and further comprising a pair of medical electrical leads 242 and 262. The medical electrical lead 242 has a lead body 244 enclosing a conductor 248 and extending from a proximal lead connector 246 to a distal lead end comprising at least one distal stimulation electrode 281, 283 and a porous mesh 280 adapted to provide fixation within the body. Similarly, the medical electrical lead 262 has a lead body 264 enclosing a conductor 268 and extending from a proximal lead connector 266 to a distal lead end comprising at least one distal stimulation electrode 272, 274 and a porous mesh 270 adapted to provide fixation within the body. Each porous mesh 280 or 270 may support or be associated with only one stimulation electrode 281, 283 or 272, 274 to concentrate the site of stimulation or more than the two schematically depicted electrodes to distribute stimulation over a wider area.

The two medical electrical leads 242 and 262 are provided in the exemplary implantable electronic stimulator system or device 20' so that the porous mesh 250 and electrodes 252, 254 can be placed in one location or position and the other porous mesh 280 and electrodes 281, 283 can be placed in one location or position selected from among the depicted electrode locations or positions 240, 242, 244, 246, 248, 250, 252, and 254. For example, the porous mesh 280 and electrodes 281, 283 can be placed at location 240 or 242 to selectively apply stimulation to the internal anal sphincter 210. The porous mesh 280 and electrodes 281, 283 can be placed in another location or position e.g., at location 244, 246, or 250 to selectively apply stimulation to the external anal sphincter 212. Alternatively, stimulation electrode 281, 283 may be placed at location 252 on, adjacent or within the levator ani 230 and the other stimulation electrodes 272, 274 may be placed at the other locations, e.g., at location 254 adjacent or within the perineal floor muscle fibers. Additional medical electrical leads may be provided to locate stimulation electrodes and mesh at additional separate locations or positions.

In one variation of the device depicted in Fig. 13, it will be understood that the control unit 22 comprises a separate pulse generator or switching circuitry that provides stimulation pulses through each medical electrical lead 242, 262, etc., to the separate locations or positions, and the conductive housing of the control unit 22 is coupled to the pulse generator circuitry to function as the indifferent or return electrode.

In another variation of the device depicted in Fig. 13, the mesh patches 250 and 280 may be coupled together as shown in the dotted lines extending between them so that continuous central portion of the mesh may be extended around the anus to locate the electrodes 272, 274 on one side of the anus, levator ani, or perineal floor and the electrodes 281, 283 on the other side of the anus, levator ani, or the perineal floor.

In any of the above variations, the implantation routes may be selected to implant electrodes 281 and 283 in operative relation to a first one of the internal anal sphincter, the external anal sphincter, the levator ani, and the pelvic floor muscle fibers and to implant electrodes 272 and 274 in operative relation with a second one different than the first one of the internal anal sphincter, the external anal sphincter, the levator ani, and the pelvic floor muscle fibers.

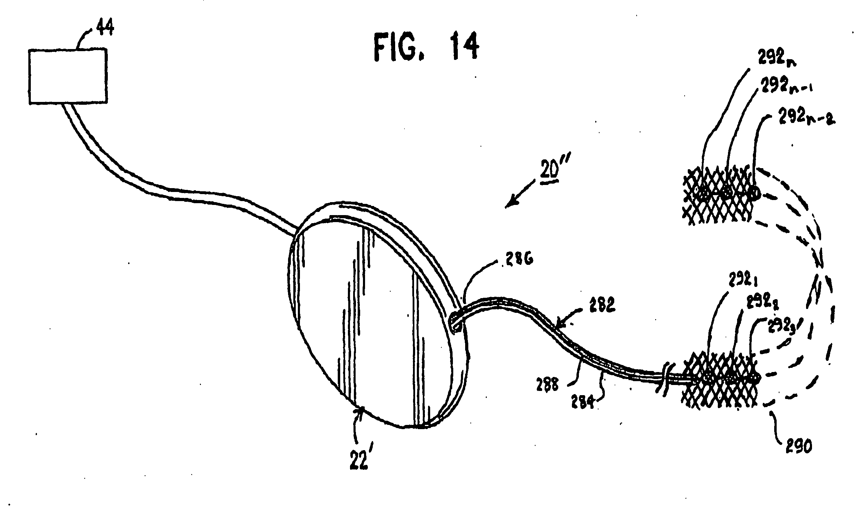

An alternative form of medical electrical lead 282 is depicted in Fig. 14 employed in an implantable electronic stimulator system or device 20" that comprises a unipolar control unit 22' having a single connector port but otherwise the same as the above-described bipolar control unit 22 and the optional fecal presence sensor 44. The medical electrical lead 282 comprises a lead body 284 enclosing a conductor 288 and extending from a proximal lead connector 286 to a distal lead end comprising a plurality N of distal stimulation electrodes 292₁ through 292ₙ extending along an elongated porous mesh 290 adapted to provide fixation within the body. The elongated mesh 290 may be of a length and width suitable for positioning around the anal cavity or the levator ani or the perineum in the locations or positions 240, 242, 244, 246, 248, 250, 252, and 254 depicted in Fig. 12. The electrodes 292₁ through 292ₙ may be arrayed linearly as depicted in Fig. 14 or in a two-dimensional array extending along the length of mesh 290. Of course, medical electrical lead 292 may be substituted for one or both of the medical electrical leads 242 and 262 in the implantable electronic stimulator system or device 20' depicted in Fig. 13.

An exemplary implantation procedure for implanting the implantable electronic stimulator system or device 20' of Fig. 13 or 20" of Fig. 14 comprises: forming a tissue pathway extending between from at least one skin incision and in relation to one of the internal anal sphincter, the external anal sphincter, and the levator ani; passing the mesh(es) and stimulation electrode(s) through the tissue pathway disposing the stimulation electrode(s) and mesh(es) in operative relation with the respective one of the internal anal sphincter, the external anal sphincter, and the levator ani; coupling the lead connector to a control unit 22 or 22'; implanting the control unit 22 and 22' within the body; and operating the control unit 22 and 22' to selectively generate electrical stimulation and to apply the electrical stimulation through the stimulation electrode(s) to the respective one of the internal anal sphincter, the external anal sphincter, and the levator ani.

Alternatively, in respect to implantable electronic stimulator system or device 20" of Fig. 14 it will be understood that the implantation route may be selected to implant electrodes 292₁, 292₂, and 292₃ in operative relation to a first one of the internal anal sphincter, the external anal sphincter, the levator ani, and the pelvic floor muscle fibers and to implant electrodes 292ₙ, 292ₙ₋₁, and 292ₙ₋₂ in operative relation with a second one different than the first one of the internal anal sphincter, the external anal sphincter, the levator ani, and the pelvic floor muscle fibers.

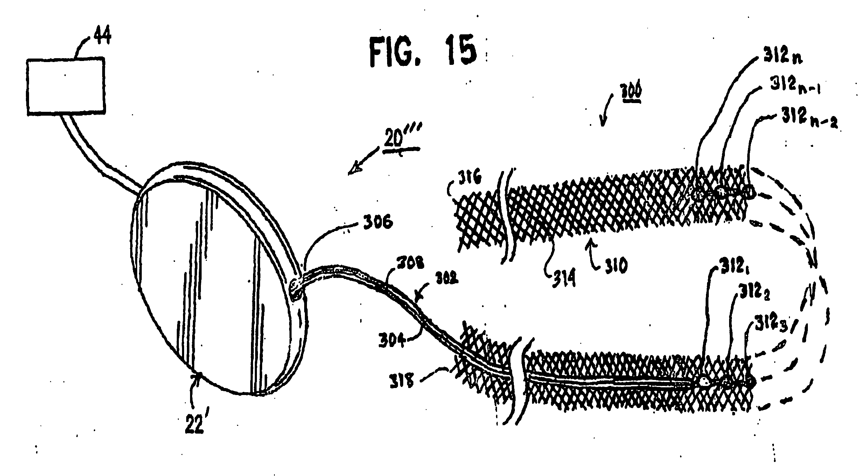

Furthermore, the medical electrical leads may be supported on a center support portion of an elongated fecal incontinence sling or fecal sling of the type described in the above-referenced U.S. Patent Application Publication No. 2007/0021650 as shown in Fig. 15. It will be understood that the term "fecal sling" encompasses any type of sling, tape, hammock or the like or a small stiff disk which is about 1 - 4 cm in length (flat, concave or convex; which can be placed under the rectum or adjacent the anus; the disk can be formed of a composite or can bioabsorbable or of a single material). The fecal sling is implanted through a tissue pathway to supports the anus in any of the manners and using any of the implantation techniques and instruments described in the prior art.

The implantable electronic stimulator system or device 20'" comprises a control unit 22' having a single connector port but otherwise the same as the above-described control unit 22, the optional fecal presence sensor 44 and a combined sling and medical electrical lead 300. The medical electrical lead 302 comprises a lead body 304 enclosing a conductor 308 and extending from a proximal lead connector 306 to a distal lead end comprising a plurality N of distal stimulation electrodes 312₁ through 312ₙ extending along a central portion of an elongated sling 310 formed of porous mesh 314. The fecal sling 310 extends between sling ends 316 and 318, and the lead body 304 extends alongside the mesh 314 through one end portion of the fecal sling 310. The elongated fecal sling 310 may be of a length and width suitable for positioning around the anal cavity or the levator ani or the perineum in the locations or positions 240, 242, 244, 246, 248, 250, 252, and 254 depicted in Fig. 12.

The electrodes 312₁ through 312ₙ may be arrayed linearly as depicted in Fig. 15 or in a two-dimensional array extending along the length of mesh 314 within the central portion of the fecal sling 310. The electrodes may also comprise electrically conductive strands of the mesh 314. Multiple stimulation electrodes may be distributed in a pattern and spacing to ensure distribution in the locations or positions extending around the anal canal 206 or the levator ani 230 or the perineal floor. The electrode(s) may be exposed on both sides of the porous mesh or insulation may be applied to the surfaces of the electrodes on one side of the mesh to enable stimulation in only one direction.

It will be understood that two sets of stimulation electrodes that are separately coupled through lead conductors to two pulse generators within control unit 22' to provide electrical stimulation through electrodes 312₁, 212₂, and 312₃ in operative relation to a first one of the internal anal sphincter, the external anal sphincter, the levator ani, and the pelvic floor muscle fibers and through electrodes 312ₙ, 312ₙ₋₁, and 312ₙ₋₂ to selected different muscles at locations or positions 240, 242, 244, 246, 248, 250, 252, and 254 depicted in Fig. 12.

An exemplary implantation procedure for implanting the implantable electronic stimulator system or device 20'" of Fig. 15 is depicted in Fig. 16. The implanting steps comprise: forming a tissue pathway extending between first and second skin incisions and posteriorly of the anus 204, the tissue pathway extending at least partly around and in proximity with the internal and external anal sphincters; passing the elongated sling 310 through the tissue pathway between the first and second skin incisions disposing the external and internal anal sphincter stimulation electrodes 312₁-312ₙ in operative relation with the respective external and internal anal sphincters; and adjusting the tension of the sling 310 applied against one or both of the interior and external anal sphincters.

The tissue pathway depicted in Fig. 16 in a female patient's body 200 extends around the urethra 234, the vagina cavity 236 of vagina 238 and around the lower part of the bowel 202 in through any of the locations or positions 240, 242, 244, 246, 248, 250, 252, and 254 depicted in Fig. 12 and described above. Of course the tissue pathway may comprise any of the tissue pathways for implanting a fecal sling described in the above-referenced U.S. Patent Application Publication No. 2007/0021650.

It will be understood that the implantation route may be selected to implant electrodes 312₁, 212₂, and 312₃ in operative relation to a first one of the internal anal sphincter, the external anal sphincter, the levator ani, and the pelvic floor muscle fibers and to implant electrodes 312ₙ, 312ₙ₋₁, and 312ₙ₋₂ in operative relation with a second one different than the first one of the internal anal sphincter, the external anal sphincter, the levator ani, and the pelvic floor muscle fibers.

The steps further include: coupling the lead connector 306 to a control unit 22'; implanting the control unit 22' within the body; and operating the control unit 22' to selectively generate electrical stimulation and to apply the electrical stimulation through the stimulation electrodes 312₁ through 312ₙ to the respective one or more of the internal anal sphincter, the external anal sphincter, and the levator ani. Of course testing of the response to electrical stimulation would be conducted to confirm the stimulation parameters set forth in Table 1.

It will be understood that the control unit 22 or 22' of Figs. 13-15 may be located subcutaneously in an abdominal location as generally indicated in Fig. 16. The sensor 44 may be located in relation to the rectum 202 to detect filling of the bowel.

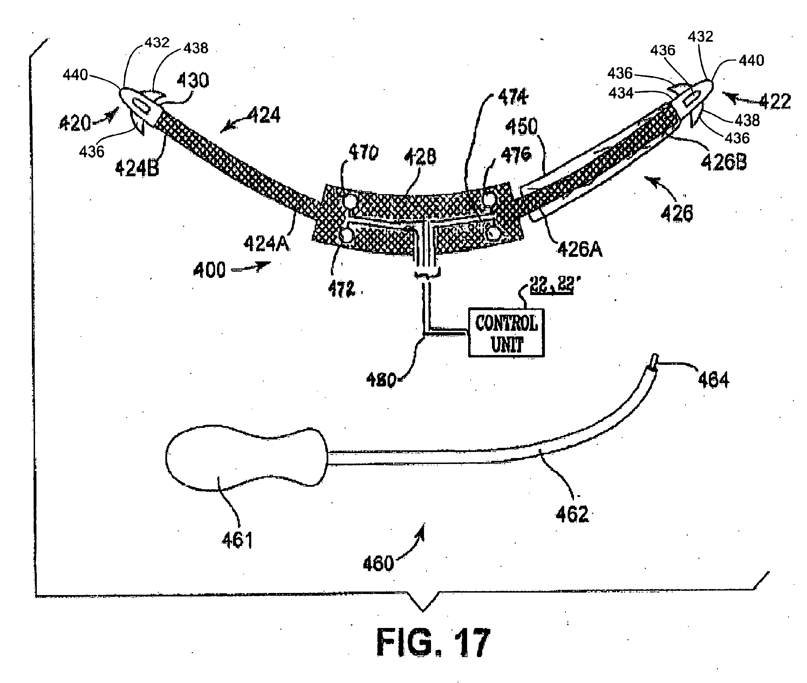

In an embodiment according to the invention illustrated in Fig. 17, the mesh patches 270, 280, and 290 or fecal sling 300 is provided with tissue anchors disposed at either or both ends to facilitate anchoring in tissue in the pelvic area without having to make obturator or suprapubic or retropubic incisions in the patient. The fecal sling 300 or mesh patches 270, 280 and 290 can be placed via a single incision made either in the perineal area or transvaginally, and then the sling or mesh can be introduced with introducers or needles. Such mesh shapes, anchor members and methods of implantation are described more fully in the above-referenced US Published Application No. 2004/0039453 A1 dated 26 February 2004 and PCT Application No. PCT/US2007/004015 filed 2 February 2007.

Referring to Fig. 17, a fecal sling 400 is illustrated that includes a first tissue anchor 420, a second tissue anchor 422, a first anchoring arm 424, a second anchoring arm 426, and a sling body ("central support portion" or "tissue support portion" 428). As illustrated, sling body 428 may be suspended between first anchoring arm 424 and second anchoring arm 426 and may be operably attached to a first end 424A, 426A of each respective arm 424, 426. Second end 424B, 426B of each anchoring arm 424, 426 is attached to respective tissue anchor 420, 422.

In addition, a set of stimulation electrodes 470, 472, 474, 476, for example, are depicted mounted to or formed as part of the central support portion 428. The stimulation electrodes are coupled either together to a single electrical conductor or through separate electrical conductors within the electrical medical lead 480 to the connector header of the control unit 22 or 22'. Stimulation generated by the control unit 22, 22' is conducted to tissue through electrodes 470, 472, 474, 476, to the electrode locations or positions 240, 242, 244, 246, 248, 250 as described above.

Tissue anchors 420, 422 are designed for anchoring the fecal sling ends (or mesh patch ends) to tissue rather than bone in an implantation procedure optionally employing instrument 460. In an exemplary embodiment, tissue anchors 420 and 422 can be placed through the incision and into tissue of the obturator foramen (e.g., the obturator internus muscle, the obturator membrane, or the obturator external muscle). Tissue anchors 420 and 422 may be driven to the desired position by the surgeon's finger or by using an insertion tool such as introducer 460.

Introducer 460 may be any type of insertion tool that can engage tissue anchor 420 to drive tissue anchor 420 through and into pelvic tissue of a desired location. Such an introducer 460 may include a durable biocompatible, curved or straight needle portion 462, made, e.g., of stainless steel, titanium, Nitinol, polymers, plastics, or other individual or combinations of materials. Handle 461 is attached at a proximal end of needle portion 462, and distal end 464 of needle portion 462 is designed to engage self-fixating tips 420 and 422, e.g., by being sized and shaped to fit within an interior channel of each tip 420, 422. Introducer 460 should have sufficient structural integrity to position tissue anchor 420 as desired. Introducer 460 may mate with or engage tissue anchor 420 by any manner, including fitting within an internal channel of a body or base of tissue anchor 420, alternately on an external portion of a body or base of a tissue anchor 420, or by interacting with fixation wings 436. Tissue anchor 420, 422 may be situated inside or outside of sleeve 450 and introducer 460.

Once a first tissue anchor 420 is placed into a desired position, a second tissue anchor 422 may be inserted through the same incision and placed in a desired position on an opposite side of the patient. As with the first tissue anchor 420, the second tissue anchor 422 may be positioned with or without the assistance of an introducer 460 and may be placed, e.g., into tissue of the obturator foramen (obturator internus muscle, obturator membrane, obturator externus muscle). Sling body 428 may be properly oriented into the desired position in relation to the urethra. It may be desirable to ensure that the sling 400 is not twisted during implantation. Positioning of implant 400 can be accomplished by selecting the point of entry and depth of each tissue anchor 420, 422.

As illustrated in Fig. 17, first and second tissue anchors 420, 422 of an implant can be substantially identical, and, as illustrated, can be described with reference to tissue anchor 420. Tissue anchors 420, 422 may also be known as anchor members, fixation members, self-fixating tips, or fasteners. The tissue anchors 420, 422 include one or more lateral extensions that can increase the force required to remove the tissue anchor from tissue after insertion into the tissue, i.e. the "pullout force." At the same time, the lateral extensions can be designed to exhibit a reduced or relatively low "insertion force," which is the amount of force used to insert the tissue anchor into tissue. The tissue anchor is designed to be essentially permanently placed upon insertion into tissue, with the single exception that if absolutely necessary to provide desired placement of the tissue anchor or an attached implant, the tissue anchor may be removed by a surgeon during an implantation procedure. The tissue anchor, and all components of the tissue anchor, can be of combined form and dimensions to result in these functional features.

In one embodiment, anchor 420 may include a body (or "base") 430 with a first (distal) end 432 and a second (proximal) end 434. A number of fixation wings (or "lateral extensions") 436 may be attached to body 430 at some point or along a length between first end 432 and second end 434. In the embodiment illustrated, anchor 420 includes four fixation wings 436 spaced evenly about a perimeter of body 430. In alternate embodiments, anchor 420 may include a greater or lesser number of fixation wings 436, positioned in any desired pattern around the body 430. Fixation wings 436 may also be referred to as or may include barbs, extensions, fins, tines, spikes, teeth, or pins.

Fixation wings 436 may according to certain embodiments be in the form of relatively thin (a thickness in the range of millimeters or less) wing-type structures that extend generally perpendicularly from the surface of body 430. Fixation wings 436 may extend away from body 430 to form a smoothly angled surface 438. Surface (or "edge") 438 may extend further from body 430 when traveling from first end 432 toward second end 434 in a continuous or other angular, curved, arcuate, concave, convex, or other pattern. The form of surface (or "edge") 438 can be one that allows for anchor 420 to be implanted through tissue in an implantation direction with reduced or minimal damage to the tissue, and reduced or minimal insertion force. Fixation wings 436 may further include tip 440. Tip 440 may be a barbed-like structure at the tail end of sloping surface 438. Tip 440 may allow for anchor 420 to resist being withdrawn from a desired anchoring position. Tip 440 may form a pointed tip 440 or may form a more rounded tip. In either case, tip 440 provides anchor 420 with a structure that helps to bind anchor 420 in a desired position in a pelvic tissue.

In alternate embodiments, fixation wing 436 may take other forms such as a barb, spike, (optionally fixed) etc., that can effectuate the implantation of anchors 420, 422 in the desired location. In addition, body 430 of anchor 420 may include barbs and spikes in addition to the fixation wing 436.

Fig. 17 includes a perspective view of one implant embodiment of the present invention, and the invention is not limited to the particular embodiment shown. It is understood that a large number of different sizes, shapes, and dimensions of implant (e.g., slings) will be suitable according to different embodiments of implants described herein. In one embodiment the sling body 428 and anchoring arms 424, 426 are all substantially one piece (i.e., "integrated") and may be of uniform width and thickness. In such an embodiment the sling may appear as one continuous ribbon or tape. In further embodiments, sling 410 may be an assembly of two or more pieces, e.g., different pieces of mesh or combinations of mesh and a biologic material.

Sling body 428 may be made by being woven, knitted, sprayed, or punched from a blank. In one aspect of the invention, sling body 428 may include one or more woven, knitted, or inter-linked filaments or fibers that form multiple fiber junctions. The fiber junctions may be formed via weaving, knitting, braiding, or through other techniques, including combinations thereof. In addition, the size of the resultant openings or pores of the mesh may be sufficient to allow tissue in-growth and fixation within surrounding tissue.

The material used to make the sling body 428, arms 424 and 426, and anchors 420 and 422, may include a variety of different plastics or other materials that are strong but conducive to being used in the body, such as, but not limited to, polypropylene, cellulose, polyvinyl, silicone, polytetrafluoroethylene, polygalactin, Silastic, carbon-fiber, polyethylene, nylon, polyester (e.g. dacron) PLLA, acetols, EPTFE and PGA. Sling body 428, arms 424 and 426, and anchors 420 and 422, each may independently be any of resorbable, absorbable or non-absorbable; optionally, some portions may be absorbable and other portions may be non-absorbable. In further embodiments the material used to make the sling body 428 may include a non-synthetic material or a synthetic and non-synthetic blend of materials. In addition, it may be preferable that the sling body 428 be relatively elastic. In other embodiments the sling may be relatively inelastic.

Some example of commercially available materials may include MarleX™ (polypropylene) available from Bard of Covington, RI, Prolene™ (polypropylene) and Mersilene (polyethylene terephthalate) Hernia Mesh available from Ethicon, of New Jersey, Gore-TeX™ (expanded polytetrafluoroethylene) available from W. L. Gore and associates, Phoenix, Arizona, and the polypropylene sling available in the SPARC™ sling system, available from American Medical Systems, Inc. of Minnetonka, Minnesota. Commercial examples of absorbable materials include Dexon™ (polyglycolic acid) available from Davis and Geck of Danbury, Connecticut, and Vicryl™ available from Ethicon.

First and second arms 424, 426 may likewise be made by weaving, knitting or in any of the other ways previously discussed in reference to sling body 428. First and second arms 424, 426 may be made of the same or different material as sling body 428 and may include the same or different physical characteristics, such as, for example, reabsorbability. In one embodiment, first and second anchoring arms 424, 426 may be a weave that results in a stronger or denser material than the weave used to make the sling body 428 so as to support more weight over a given surface area. In one embodiment the arms 424, 426 may not be woven. In further embodiments, sling body 428 and the first and second arms 424, 426 may be made of one continuous weave structure of the same or different weave densities.

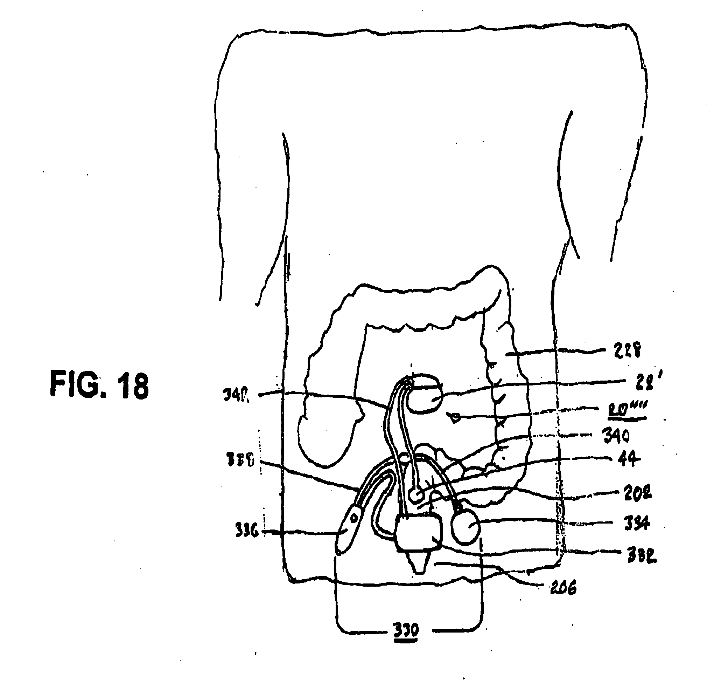

Referring to FIG. 18, the abdominal region of a patient is depicted schematically, exposing the intact lower digestive tract 228 terminating at the anal canal 206 surrounded by the anal sphincter. In this illustrated example, an artificial anal sphincter 330 has been implanted in the abdominal region of patient that obscures the internal and external anal sphincters 210 and 212. An implantable electronic stimulator system or device 20"" is also depicted in Fig. 18. In this example, a set of stimulation electrodes, e.g., electrodes 312₁ through 312ₙ on the surface of the inflatable/deflatable cuff of the artificial anal sphincter. The cuff is typically implanted around the external anal sphincter 212, and the electrodes 312₁ through 312ₙ on the inner side of the cuff are thereby be disposed in location or position 250 of Fig. 12 facing toward the external and internal anal sphincters 212 and 210.

The artificial anal sphincter 330 may comprise the Acticon® Neosphincter that simulates normal sphincter function to give the patient control over defecation. The inflatable cuff 332 is depicted implanted around a segment of the anal sphincter surrounding the anal canal 206 to occlude the anal canal 206 when the cuff 332 is inflated. A pressure regulating balloon/inflation fluid source or simply balloon 334 is implanted in the prevesical space. A manually activated pump 336 is adapted to be implanted in the scrotum of the male patient. Tubes 338 and 340 interconnect the interior fluid chambers of the cuff 332, balloon 334, and the pump 336 for fluid transfer therebetween.

The amount of fluid in the balloon 334 and cuff 332 controls the amount of pressure exerted by the cuff 332 against the anal canal to inhibit a bowel movement. The pump 336 features a deactivation option so that the cuff 332 can be deflated for a prolonged period of time, and a septum port so that fluid can be added percutaneously to the artificial anal sphincter 330 using a syringe. The lower part of the pump 336 is soft and squeezable, whereas the upper part containing the deactivation button is hard. The deactivation button can be felt on the upper, hard part of the pump and depressed to activate or deactivate the pump 336. The septum port can be felt at the tip of the lower, soft part of the pump 336 and allows the physician to add additional fluid, if needed, without surgery.

The control unit 22' generates stimulation pulses that are delivered through medical electrical lead 342 to the array of stimulation electrodes on cuff 332. The control unit 22' is coupled to a pressure sensor 44 disposed in relation to the lower part of the rectum to provide a signal to the control unit circuitry that processes the signal to detect filling of the rectum. The generation of stimulation pulses may be made dependent on sensing pressure in the rectum and may be correlated to the patient's operation of the deactivation option.

In each control unit 22 and 22', control unit logic and/or algorithms are provided to analyze the signal output by the sensor 44 or 160 so as to distinguish between: (a) a first signal, indicative of imminent fecal incontinence, and (b) a second signal, indicative of voluntary voiding by the patient. For example, the control unit logic and/or algorithms may be adapted to distinguish between the first and second signals responsive to a rate of change of the signal generated by the sensor. Alternatively or additionally, the control unit logic and/or algorithms are adapted to gather information regarding the signal over an extended period and to analyze the information to find a pattern characteristic of the patient, for use in determining when imminent fecal incontinence is likely. In this case, the control unit logic or algorithms are typically adapted to associate with the pattern a time-varying threshold to which a level of the signal is compared.

It will be appreciated by persons skilled in the art that the present invention is not limited to what has been particularly shown and described hereinabove.

It will be understood that certain of the above-described structures of the above-described preferred embodiments are not necessary to practice the present invention and are included in the description simply for completeness of an exemplary embodiment or embodiments. It will also be understood that there may be other structures that are not disclosed and are not necessary to the practice of the present invention.

## Claims

1. A device for treating fecal incontinence in a body of a mammal having a rectum (202) formed of a rectal wall (208) extending to an anus (204), wherein the normal rectal wall includes an anal sphincter muscle comprising an internal anal sphincter (210) surrounding the anus and an external anal sphincter (212) surrounding the internal anal sphincter, and the pelvic floor comprises a levator ani (230) supporting the rectum and subcutaneous pelvic floor muscle fibers (218), the device comprising:
a device body comprising an elongated fecal sling (400) extending between first and second device ends (424A, 426A) and including a central support portion adapted to be positioned in supportive relation to one or more of the internal anal sphincter surrounding the anus, the external anal sphincter surrounding the internal anal sphincter, and the pelvic floor;
a medical electrical lead (480) having a lead body extending from a proximal lead connector to a distal lead end comprising at least one distal stimulation electrode supported by the central support portion; and
self-fixating tissue anchors (420, 422) coupled to the first and second ends, the first and second ends each having a shape that facilitates insertion into tissue to tension the device body and that resists withdrawal from the penetrated tissue.

2. The device of claim 1, wherein:
the device body comprises extension portions (424, 426); and
the self-fixating tissue anchors are each connected to one of the extension portions and each anchor further comprises:
a base (430) comprising a proximal base end (434) and a distal base end (432), the proximal base end being connected to the extension portion: and
at least one fixed lateral extension (436) extending from the base, the lateral extension comprising a lateral extension body comprising boundaries that include a leading edge, a trailing edge, and a length at which the lateral extension meets the base, wherein the trailing edge has a thickness greater than the leading edge,
wherein optionally the extension portion includes at least one stimulation electrode.

3. The device of claim 2, wherein each of the self-fixating tissue anchors includes two or more of the lateral extensions, all lateral extensions extending in a different direction from locations that are at the same length-wise position of the base.

4. In combination, the device according to any of the claims 1-3 and an insertion tool (460), the insertion tool comprising a handle (461) and a needle (462) extending from the handle, the needle comprising a proximal end attached to the handle and a distal end, the distal end (464) comprising a needle tip that engages one of the self-fixating tissue anchors to insert the self-fixating tissue anchor into tissue.

5. The combinations of claim 4 wherein:
when the needle distal end is engaged with one of the self-fixating tissue anchors, the self-fixating tissue anchor maintains an orientation relative to the insertion tool with lateral extensions aligned for implantation into fibrous tissue so the lateral extensions penetrate the fibrous tissue in an orientation that is non-parallel to the direction of fibers of the fibrous tissue.

6. The device of claim 1, wherein the elongated fecal sling comprises a porous mesh.

7. The device of claim 6, wherein:
the mesh comprises electrically conductive strands (314); and
the at least one stimulation electrode comprises the electrically conductive strands.

8. The device of claim 1, wherein the at least one stimulation electrode comprises a plurality of stimulation electrodes (470, 472; 474, 476) each attached to the elongated fecal sling.

9. The device of claim 8, wherein:
the elongated fecal sling comprises first and second opposing sides; and
each of the stimulation electrodes comprises a first side corresponding to the first side of the sling and a second side corresponding to the second side of the sling, the first side of each stimulation electrode is exposed to conduct an electrical signal, and the second side of each stimulation electrode is electrically insulated.

## Patentansprüche

1. Vorrichtung zur Behandlung von Stuhlinkontinenz in einem Körper eines Säugers mit einem Rektum (202), das aus einer Rektalwand (208) gebildet ist, die sich zu einem Anus (204) erstreckt, wobei die normale Rektalwand einen Analsphinktermuskel mit einem inneren Analsphinkter (210), der den Anus umgibt, und einem äußeren Analsphinkter (212) aufweist, der den inneren Analsphinkter umgibt, und der Beckenboden einen Anusheber (230) aufweist, der das Rektum und subkutane Beckenboden-Muskelfasern (218) abstützt, wobei die Vorrichtung aufweist:
einen Vorrichtungskörper mit einer länglichen Fäkalschlinge (400), die sich zwischen einem ersten und einem zweiten Vorrichtungsende (424A, 426A) erstreckt und einen Mittelstützabschnitt aufweist, der geeignet ist, in Stützbeziehung zum inneren Analsphinkter, der den Anus umgibt, äußeren Analsphinkter, der den inneren Analsphinkter umgibt, und/oder Beckenboden positioniert zu werden;
eine medizinische elektrische Leitung (480) mit einem Leitungskörper, der sich von einem proximalen Leitungsverbinder zu einem distalen Leitungsende mit mindestens einer distalen Stimulationselektrode erstreckt, die durch den Mittelstützabschnitt abgestützt wird; und
selbstfixierende Gewebsanker (420, 422), die mit dem ersten und zweiten Ende gekoppelt sind, wobei das erste und zweite Ende jeweils eine Form haben, die Einführen in Gewebe erleichtert, um den Vorrichtungskörper zu spannen, und die Herausziehen aus dem penetrierten Gewebe widersteht.

2. Vorrichtung nach Anspruch 1, wobei:
der Vorrichtungskörper Verlängerungsabschnitte (424, 426) aufweist; und
die selbstfixierenden Gewebsanker jeweils mit einem der Verlängerungsabschnitte verbunden sind und jeder Anker ferner aufweist:
eine Basis (430) mit einem proximalen Basisende (434) und einem distalen Basisende (432), wobei das proximale Basisende mit dem Verlängerungsabschnitt verbunden ist; und
mindestens eine feste Seitenverlängerung (436), die sich von der Basis erstreckt, wobei die Seitenverlängerung einen Seitenverlängerungskörper mit Grenzen aufweist, die eine Vorderkante, eine Hinterkante und eine Länge aufweisen, an der die Seitenverlängerung mit der Basis zusammentrifft, wobei die Hinterkante eine größere Dicke als die Vorderkante hat,
wobei optional der Verlängerungsabschnitt mindestens eine Stimulationselektrode aufweist.

3. Vorrichtung nach Anspruch 2, wobei jeder der selbstfixierenden Gewebsanker zwei oder mehr Seitenverlängerungen aufweist, wobei sich alle Seitenverlängerungen in einer unterschiedlichen Richtung von Stellen aus erstrecken, die an der gleichen Längsposition der Basis liegen.

4. Kombination aus der Vorrichtung nach einem der Ansprüche 1 bis 3 und einem Einführwerkzeug (460), wobei das Einführwerkzeug einen Griff (461) und eine sich vom Griff erstreckende Nadel (462) aufweist, die Nadel ein am Griff angebrachtes proximales Ende und ein distales Ende aufweist und das distale Ende (464) eine Nadelspitze aufweist, die einen Eingriff mit einem der selbstfixierenden Gewebsanker herstellt, um den selbstfixierenden Gewebsanker in Gewebe einzuführen.

5. Kombinationen nach Anspruch 4, wobei:
bei Eingriff des distalen Nadelendes mit einem der selbstfixierenden Gewebsanker der selbstfixierende Gewebsanker eine Orientierung relativ zum Einführwerkzeug beibehält, wobei Seitenverlängerungen zur Implantation in Fasergewebe so ausgerichtet sind, dass die Seitenverlängerungen das Fasergewebe in einer Orientierung penetrieren, die zur Richtung von Fasern des Fasergewebes nicht parallel ist.

6. Vorrichtung nach Anspruch 1, wobei die längliche Fäkalschlinge ein poröses Maschenmaterial aufweist.

7. Vorrichtung nach Anspruch 6, wobei:
das Maschenmaterial elektrisch leitende Litzen (314) aufweist; und
die mindestens eine Stimulationselektrode die elektrisch leitenden Litzen aufweist.

8. Vorrichtung nach Anspruch 1, wobei die mindestens eine Stimulationselektrode mehrere Stimulationselektroden (470, 472, 474, 476) aufweist, die jeweils an der länglichen Fäkalschlinge angebracht sind.

9. Vorrichtung nach Anspruch 8, wobei:
die längliche Fäkalschlinge eine erste und eine zweite gegenüberliegende Seite aufweist; und
jede der Stimulationselektroden eine erste Seite in Entsprechung zur ersten Seite der Schlinge und eine zweite Seite in Entsprechung zur zweiten Seite der Schlinge aufweist, die erste Seite jeder Stimulationselektrode freiliegt, um ein elektrisches Signal zu leiten, und die zweite Seite jeder Stimulationselektrode elektrisch isoliert ist.

## Revendications

1. Dispositif pour le traitement de l'incontinence fécale sur un corps de mammifère avec un rectum (202) constitué d'une paroi rectale (208) s'étendant vers un anus (204), la paroi rectale normale comportant un muscle de sphincter anal avec un sphincter anal interne (210) entourant l'anus et un sphincter anal externe (212) entourant le sphincter anal interne, et le plancher pelvien comportant un levator ani (230) supportant le rectum et des fibres musculaires (218) de plancher pelvien sous-cutanées, ledit dispositif comprenant :
un corps de dispositif comportant une élingue fécale (400) oblongue qui s'étend entre une première et une deuxième extrémités (424A, 426A) de dispositif et présentant une partie centrale de support prévue pour être positionnée en relation de support avec un ou plusieurs muscles parmi le sphincter anal interne entourant l'anus, le sphincter anal externe entourant le sphincter anal interne, et le plancher pelvien ;
une dérivation électrique médicale (480) avec un corps de dérivation s'étendant d'un connecteur proximal de dérivation à une extrémité distale de dérivation, comportant au moins une électrode de stimulation distale supportée par la partie central du support ; et
des ancrages de tissu auto-fixants (420, 422) reliés à la première et à la deuxième extrémités, la première et la deuxième extrémités présentant chacune une forme facilitant l'insertion dans un tissu pour tendre le corps de dispositif, et la résistance à un retrait du tissu d'insertion.

2. Dispositif selon la revendication 1 où :
le corps de dispositif comprend des parties d'extension (424, 426) ; et
où les ancrages de tissu auto-fixants sont reliés chacun à une des parties d'extension et où chaque ancrage comporte en outre :
une base (430) avec une extrémité de base proximale (434) et une extrémité de base distale (432), l'extrémité de base proximale étant reliée à la partie d'extension : et
au moins une extension latérale de fixation (436) s'étendant depuis la base, ladite extension latérale comportant un corps latéral d'extension avec des limites présentant un bord d'attaque, un bord postérieur, et une longueur de l'extension latérale à la base, le bord postérieur ayant une épaisseur supérieure au bord d'attaque, la partie d'extension comportant facultativement au moins une électrode de stimulation.

3. Dispositif selon la revendication 2, où chaque ancrage de tissu auto-fixant comprend au moins deux extensions latérales, toutes les extensions latérales s'étendant dans des directions différentes depuis des points situés au même emplacement sur la longueur de la base.

4. Combinaison d'un dispositif selon l'une des revendications 1 à 3 et d'un outil d'insertion (460), l'outil d'insertion comportant une poignée (461) et une aiguille (462) s'étendant depuis la poignée, l'aiguille présentant une extrémité proximale fixée à la poignée et une extrémité distale, l'extrémité distale (464) présentant une pointe d'aiguille en prise avec un des ancrages de tissu auto-fixants pour introduire ledit ancrage de tissu auto-fixant dans un tissu.

5. Combinaison selon la revendication 4, où :
quand l'extrémité distale d'aiguille est en prise avec un des ancrages de tissu auto-fixants, ledit ancrage de tissu auto-fixant conserve une orientation par rapport à l'outil d'insertion où les extensions latérales sont alignées pour implantation dans un tissu fibreux, de telle manière que les extensions latérales pénètrent dans le tissu fibreux suivant une orientation non parallèle à la direction des fibres du tissu fibreux.

6. Dispositif selon la revendication 1, où l'élingue fécale oblongue est constituée d'un treillis poreux.

7. Dispositif selon la revendication 6, où :
le treillis est constitué de fils conducteurs électriques (314) ; et
où l'électrode ou les électrodes de stimulation comprennent les fils conducteurs électriques.

8. Dispositif selon la revendication 1, où l'électrode ou les électrodes de stimulation comportent une pluralité d'électrodes de stimulation (470, 472 ; 474, 476) respectivement fixées à l'élingue fécale oblongue.

9. Dispositif selon la revendication 8, où :
l'élingue fécale oblongue présente un premier et un deuxième côtés opposés ; et
où chaque électrode de stimulation présente un premier côté correspondant au premier côté de l'élingue, et un deuxième côté correspondant au deuxième côté de l'élingue, le premier côté de chaque électrode de stimulation étant exposé pour conduire un signal électrique, et le deuxième côté de chaque électrode de stimulation étant isolé électriquement.
